# EUROPÄISCHE PATENTSCHRIFT

(11) **EP 2 644 095 B1**
(45) Veröffentlichungstag und Bekanntmachung des Hinweises auf die Patenterteilung: **09.09.2015**
(21) Anmeldenummer: 13161550.2
(22) Anmeldetag: 28.03.2013
(51) Int. Cl.: A61B 6/00, G01N 23/04

(54) **Computertomographiesystem und Verfahren zur Datenermittlung für eine Störeinfluss-korrigierte Computertomographieaufnahme eines Untersuchungsobjekts**
Computertomograph and method for determining data for a computertomographic scan of an object, corrected for perturbations
Système de tomodensitométrie et procédé de détermination de dates pour un enregistrement tomodensitométrique à perturbation corrigée d'un objet d'étude

(30) Priorität: 30.03.2012 DE 102012205225
(43) Veröffentlichungstag der Anmeldung: 02.10.2013
(73) Patentinhaber: Fraunhofer-Gesellschaft zur Förderung der angewandten Forschung e.V., 80686 München (DE)
(72) Erfinder: Schmitt, Michael, 91091 Großenseebach (DE); Reisinger, Stefan, 90763 Fürth (DE); Voland, Virginia, 90763 Fürth (DE); Salamon, Michael, 90763 Fürth (DE); Krumm, Michael, 88662 Überlingen (DE)
(74) Vertreter: Hersina, Günter

(56) Entgegenhaltungen:
- DE-A1-102007 023 925
- FRANK NACHTRAB ET AL: "Progress in sub-micrometer resolution computed tomography", NUCLEAR SCIENCE SYMPOSIUM CONFERENCE RECORD, 2008. NSS '08. IEEE (19-25 OCT. 2008), IEEE, PISCATAWAY, NJ, USA, 19. Oktober 2008 (2008-10-19), - 25. Oktober 2008 (2008-10-25), Seiten 532-535, XP031419513, ISBN: 978-1-4244-2714-7
- ALEXANDER SASOV ET AL: "Compensation of mechanical inaccuracies in micro-CT and nano-CT", PROCEEDINGS OF SPIE, Bd. 7078, 28. August 2008 (2008-08-28), Seiten 70781C-70781C-9, XP055066557, ISSN: 0277-786X, DOI: 10.1117/12.793212
- SALMON P L ET AL: "A post-scan method for correcting artefacts of slow geometry changes during micro-tomographic changes", JOURNAL OF X-RAY SCIENCE AND TECHNOLOGY, IOS PRESS, AMSTERDAM, NL, Bd. 17, 1. Januar 2009 (2009-01-01), Seiten 161-174, XP009170377, ISSN: 0895-3996, DOI: 10.3233/XST-2009-0220

## Beschreibung

Die vorliegende Erfindung bezieht sich auf ein Computertomographiesystem und ein Verfahren zur Datenermittlung für eine Computertomographieaufnahme eines Untersuchungsobjekts bzw. Prüfkörpers. Insbesondere bezieht sich die vorliegende Erfindung auf eine Vorrichtung und ein Verfahren zur einsatzsynchronen Detektion und Kompensation von zeitabhängigen Störeinflüssen an Computertomographiesystemen.

Die Computertomographie (CT) wird auf dem Gebiet der Medizin als auch beispielsweise der zerstörungsfreien Materialprüfung weit verbreitet eingesetzt. Bei der Computertomographie wird die Wechselwirkung zwischen der erzeugten Röntgenstrahlung und der Materie bzw. dem Material des Untersuchungsobjekts erfasst.

Bei üblichen Computertomographiesystemen wird von einer Röntgenröhre als Strahlungsquelle Röntgenstrahlung in Form eines Fächerstrahls oder eines Kegelstrahls ausgehend von einem Brennfleck (Fokus) der Röntgenröhre abgestrahlt, durchdringt das Untersuchungsobjekt und fällt auf einen Röntgendetektor, z.B. ein Flachbilddetektor. Entsprechend dem Prinzip der CT (CT = Computertomographie) trifft Röntgenstrahlung auf Materie, wobei je nach Material- und Objektbeschaffenheit, z.B. Materialdichte und Durchstrahlungslängen, ein unterschiedlich großer Anteil der Strahlung von dem Untersuchungsobjekt absorbiert wird. Ein einzelnes Durchstrahlungsbild entsteht nun aus der Detektion und Visualisierung nicht absorbierter Röntgenstrahlung. Aufgrund der Überlagerung von Bereichen unterschiedlicher Dichte enthält eine solche Projektion allerdings keine Tiefeninformationen hinsichtlich des Untersuchungsobjekts. Erst durch die Durchstrahlung bzw. Projektion der Fläche des Untersuchungsobjekts unter weiteren Winkelpositionen lässt sich eine räumliche Information des Untersuchungsobjekts ermitteln. Je mehr Projektionen unter unterschiedlichen Winkeln aufgenommen werden, umso mehr Tiefeninformationen (3D-Informationen) liegen vor, die man dann aus den Aufnahmen "rekonstruieren" kann. Anhand der Informationen des resultierenden Durchstrahlungsdatensatzes (Projektionsdatensatz) aus einer Vielzahl von Durchstrahlungsaufnahmen wird nun beispielsweise mittels einer mathematischen Transformation jeweils einem Volumenelement (Voxel) des Untersuchungsobjekts ein Schwächungswert zugeordnet. Die Auflösung des rekonstruierten Volumens, d.h. der 2D- oder 3D-Darstellung des Untersuchungsobjekts hängt dabei signifikant von der Anzahl der an unterschiedlichen Winkelpositionen durchgeführten Durchstrahlungsaufnahmen des Untersuchungsobjekts sowie von der jeweiligen Größe der Sensorelemente des Röntgendetektors ab.

Für eine möglichst artefaktfreie Rekonstruktion von Schichtinformationen des Untersuchungsobjekts bzw. Prüfkörpers in der digitalen 2D- oder 3D-Computertomographie ist nun eine möglichst genaue Kenntnis der Aufnahmegeometrie der strahlungsemittierenden Anordnungen in Bezug auf das Untersuchungsobjekt und den Röntgenstrahlungsdetektor erforderlich. Die erreichbare Ortauflösung, d.h. die 2D-/3D-Wiedergabegenauigkeit, und die resultierende Bildschärfe hängen nun von den geometrischen Eigenschaften des bildgebenden Systems bestehend aus der Röntgenröhre und dem Röntgendetektor sowie der Justage der Komponenten zueinander und zu der Manipulationseinheit des zu untersuchenden Objekts ab. In der Fächerstrahl- oder Kegelstrahlgeometrie eines CT-Systems ist es nun für eine möglichst artefaktfreie Rekonstruktion von Bildinformationen insbesondere erforderlich, dass die genaue Position der Röntgenstrahlenquelle, d.h. des Brennflecks der Röntgenröhre (Fokus), in alle drei Raumrichtungen bekannt ist.

In Abhängigkeit von der Größe des Brennflecks bzw. Fokus werden unterschiedliche Bezeichnungen für den Brennfleck verwendet. Diesbezüglich wird auf die nachfolgende Tabelle 1 verwiesen.

**Tabelle 1: Fokusbezeichnung nach Brennfleckgröße. Für die Beschleunigungsspannungen sind typische Richtwerte angegeben. [Quelle: Gevatter, Grünhaupt, Springer, 2006]**

| **Bezeichnung** | **Beschleunigungsspannung /kV** | **Fokusgröße / µm** |
|---|---|---|
| Standardfokus | 450 | 500-800 |
| Minifokus | 250-350 | 100-300 |
| Mikrofokus | Bis 230 | 5-20 |
| Nanofokus | 50-90 | 1-8 |

Für eine ausreichende Bildqualität wird nun angenommen, dass die Position des Brennflecks mit einer Genauigkeit von ±0,1*v_{eff} bekannt sein sollte. Die effektive Voxelgröße v_{eff} (bezüglich einer Voxelkante) im Bild hängt dabei von der geometrischen Voxelgröße und der Ortsauflösung des bildgebenden Systems ab, welche wiederum abhängig von der Vergrößerung der Abbildung durch die Ausdehnung der Strahlenquelle und durch die Streueigenschaften des Röntgendetektors beeinflusst werden kann. Daher ist für eine möglichst Störeinfluss-freie Computertomographieaufnahme anzunehmen, dass die Brennfleckposition insbesondere mit einer Genauigkeit deutlich unterhalb der Brennfleckgröße bekannt sein sollte.

Im Fall von hochauflösenden CT-Messungen, bei denen kleine Brennfleckgrößen (Mikrofokus oder Nanofokus, vgl. Tabelle 1) eingesetzt werden, ist es jedoch nicht immer gegeben, dass die Brennfleckposition mit einer Genauigkeit deutlich unterhalb der Brennfleckgröße bekannt ist. Aufgrund thermischer Einflüsse und durch eine mögliche röhreninteme Fokussierung ändert sich die Brennfleckposition über der Zeit. Temperaturänderungen der Röntgenquelle während der Datenerfassung (Datenakquisition) können durch eine Verschiebung der Brennfleckposition in Abbildungsrichtung zudem zu einer Veränderung der Vergrößerung in der Abbildung und somit zu inkonsistenten Durchstrahlungsaufnahmen bzw. Projektionsdaten führen. Die Stabilität der Brennfleckposition kann durch ein ausführliches Warmfahren (warm up) der Röntgenröhre begünstigt werden. Erfahrungen zeigen jedoch, dass eine ausreichende Stabilität der Position des Brennflecks über der Dauer einer kompletten Messung von im Allgemeinen mehreren Hundert Durchstrahlungsaufnahmen (Projektionen) nicht in ausreichendem Maß sichergestellt werden kann.

Wird nun trotz örtlicher Schwankungen des Brennflecks eine konstante Brennfleckposition angenommen, entstehen in der Rekonstruktion der Daten sogenannte Bewegungsartefakte. Diese Artefakte reduzieren die Auflösung bzw. die Detailerkennbarkeit der Messung in dem resultierenden 2D- oder 3D-CT-Bild. Durch Verschmierungen im Bild können Strukturgrößen unterhalb der durch die Bewegung des Brennflecks bedingten Geometrieänderung nicht mehr aufgelöst werden. Dies führt zu ungenauen Analyse- und Messergebnissen. Unter der Annahme einer konstanten Brennfleckposition können daher nur dann Rekonstruktionsdaten ausreichender Bildqualität erzeugt werden, falls sich die Position des Brennflecks während der Datenakquisition innerhalb der oben definierten Toleranzgrenze bewegt, die deutlich unterhalb der erforderlichen Auflösung liegt. Da diese Toleranzgrenze im Falle hochauflösender Messungen sehr gering ist, sind weitere Maßnahmen erforderlich, um während der Datenerfassung die Brennfleckposition bzw. dessen etwaige Bewegung zu bestimmen und diese ermittelte Bewegung dann bei der Rekonstruktion der Durchstrahlungsdatensätze (Projektionsdatensätze) zur Fehlerkompensation zu berücksichtigen. Eine solche Vorgehensweise zur Detektion und zum Ausgleich der Brennfleckbewegung sollte einerseits möglichst mit relativ geringem zusätzlichen Aufwand eine verbesserte Bildqualität erzeugen. Hierzu ist es auch erforderlich, dass relativ präzise Werte der Brennfleckposition zur Kompensation der Brennfleckbewegung bestimmt werden.

Die wissenschaftliche Veröffentlichung von Nachtrab, F. [et al.] "Progress in submicrometer resolution computed tomography" [in: IEEE Nuclear Science Symposium Conference Record. 2008, NSS '08. IEEE (19-25 OCT. 2008), IEEE, PISCATAWAY, NJ, USA, 19. Oktober 2008 (2008-10-19), - 25. Oktober 2008 (2008-10-25), Seiten 532-535, XP031419513, ISBN: 978-1-4244-2714-7] bezieht sich auf den Fortschritt in der Computertomographie mit Sub-Mikrometer-Auflösung. Dort wurde festgestellt (vgl. Abschnitt II - D), dass eine Verschiebung der Brennpunktposition insbesondere während langer Messzeitdauern bei CT-Systemen stattfindet. Eine Unschärfe (blurring) der Bilder, die durch diese Verschiebung hervorgerufen wird, kann vermieden werden, indem mehrere Bilder von kürzeren Belichtungszeiten aufgenommen werden und diese dann später aufsummiert werden, unter Verwendung eines digitalen Bildkorrelationsalgorithmus, um die Verschiebung zu korrigieren, die zwischen zwei aufeinanderfolgenden Bildern aufgetreten ist.

Die wissenschaftliche Veröffentlichung von Salmon et al. "A post-scan method for correcting artefacts of slow geometry changes during micro-tomographic changes" [JOURNAL OF X-RAY SCIENCE AND TECHNOLOGY, IOS PRESS, AMSTERDAM, NL, Bd. 17, 1. Januar 2009 (2009-01-01), Seiten 161-174, XP009170377, ISSN: 0895-3996, DOI: 10.3233/XST-2009-0220] bezieht sich auf ein Verfahren nach Abschluss eines Kennvorgangs zum Korrigieren von Artefakten aufgrund von langsamen Geometrieänderungen während mikrotomographischer Aufnahmen. Dabei wird ein kompletter Satz an Referenz-Durchstrahlungsaufnahmen nach Abschluss des Messverfahrens aufgenommen.

Ausgehend von diesem Stand der Technik besteht die der vorliegenden Erfindung zugrundeliegende Aufgabe darin, ein verbessertes Konzept zur Datenermittlung für eine möglichst Störeinfluss-reduzierte Computertomographieaufnahme eines Untersuchungsobjekts bzw. Prüfkörpers in einem Computertomographiesystem zu schaffen.

Diese Aufgabe wird durch ein Verfahren zur Datenermittlung für eine Computertomographieaufnahme gemäß Anspruch 1 und durch eine Computertomographiesystem zur Datenermittlung für eine CT-Aufnahme gemäß Anspruch 12 gelöst.

Der Kerngedanke der vorliegenden Erfindung besteht nun darin, dass die Bestimmung von Störeinflüssen durch ein mehrmaliges Akquirieren der gleichen Durchstrahlungsdaten (Projektionsdaten) in Form von Referenz-Durchstrahlungsaufnahmen (Referenzprojektionen) in den Messablauf integriert wird. Während der Akquisition des Durchstrahlungsdatensatzes (Projektionsdatensatzes) des Untersuchungsobjekts werden eine oder mehrere zuvor definierte Referenzpositionen von der Rotationsachse und ggf. weiteren Achsen, um die das Untersuchungsobjekt hinsichtlich des CT-Systems bewegbar bzw. relativ zueinander rotierbar ist, angefahren, um dort Durchstrahlungsaufnahmen (Projektionen) des Untersuchungsobjekts zu erfassen, welche als Referenz zur Bestimmung von Störeinflüssen herangezogen werden können. Ohne eine geometrische Veränderung der Abbildung oder sonstiger Störeinflüsse entstehen so bis auf ein Bildrauschen identische Referenz-Durchstrahlungsaufnahmen. Durch entsprechende Verarbeitungsverfahren, z.B. unter Verwendung einer Kreuzkorrelation oder dem Verfahren der kleinsten Quadrate oder auch anderer denkbarer Bildverarbeitungsverfahren, werden Änderungen in der Abbildungsgeometrie zwischen der Röntgenröhre, dem Untersuchungsobjekt und/oder dem Röntgendetektor bzw. oder auch sonstige Störeinflüsse detektiert. Die aktuell aufgenommene Referenz-Durchstrahlungsaufnahme wird dafür in Bezug zur der zuletzt aufgenommenen, der zuerst aufgenommenen oder einer beliebigen anderen Referenz-Durchstrahlungsaufnahme an derselben Achsenposition (Referenz-Winkelposition) gesetzt.

Resultierende Abbildungsänderungen, die über ein Bildrauschen hinausgehen und auf Änderungen in der Abbildungsgeometrie auf andere Störeinflüsse zurückzuführen sind, können nun so "zeitabhängig" detektiert werden, da unterschiedliche Referenzinformationen hinsichtlich des jeweiligen Störeinflusses bzw. dessen zeitlichen Verlaufs zu unterschiedlichen Zeitpunkten vorliegen. Abhängig von dem gewählten Abstand der Referenzzeitpunkte zum Erfassen einer oder mehrerer Referenz-Durchstrahlungsaufnahmen oder von der Anzahl der aufgenommenen Referenz-Durchstrahlungsaufnahmen, die beispielsweise auch als sogenannte Stützstellen für eine Interpolation zur Ermittlung einer Korrekturfunktion herangezogen werden können, kann eine hohe zeitliche Abtastung und damit einhergehend die Detektion auch zeitlich hochfrequenter Störeinflüsse erreicht werden.

Ausführungsbeispiele der vorliegenden Erfindung beschreiben ein Verfahren zur Erkennung und Kompensation von Brennfleckbewegungen basierend auf den Bilddaten des Untersuchungsobjekts ohne den Einsatz von Referenzobjekten. Die gewonnenen Informationen über die Brennfleckbewegung werden als Informationen über die Abbildungsgeometrie für die Rekonstruktion der Bilddaten verwendet, ohne mechanische Bewegungen durchzuführen oder Bildverarbeitungsoperatoren auf den Projektionsdaten anzuwenden. Somit kann der untersuchbare Objektbereich und ferner die erreiche Ortsauflösung maximiert werden, wobei gemäß Ausführungsbeispielen der vorliegenden Erfindung ein Verfahren und eine Vorrichtung zur Kompensation von Brennfleckwanderungen zum Einsatz kommen, die ohne den Einsatz von Referenzobjekten und ohne mechanische Bewegung der Röntgenkomponenten auskommen.

Die vorliegende Vorgehensweise zur Datenermittlung für eine möglichst Störeinfluss-reduzierte Computertomographieaufnahme eines Untersuchungsobjekts in einem CT-System wird nun beispielsweise im Fall einer CT-Messung mit k Winkelschritten (k ≥ z.B. 360, 720, 800, 1600 oder beliebige Zwischenwerte) den folgenden Ablauf aufweisen. Zunächst werden eine oder mehrere aussagekräftige Referenzpositionen ermittelt bzw. definiert. Solche Referenzwinkelpositionen ermöglichen, dass basierend auf den Referenz-Durchstrahlungsaufnahmen an diesen Referenz-Winkelpositionen aussagekräftige Referenzinformationen erhalten werden können, die ableitbare Informationen hinsichtlich des Störeinflusses auf die Mess-Durchstrahlungsaufnahmen liefern können. Dabei werden beispielsweise Referenz-Winkelpositionen gewählt, die Referenz-Durchstrahlungsaufnahmen ermöglichen, die hervorgehobene, klare äußere Strukturen, einen hohen Bildkontrast, ein günstiges Aspektverhältnis, z.B. für möglichst kurze Durchstrahlungslängen, und/oder interne Strukturen bzw. Strukturierungen deutlich zeigen. Damit können unterschiedliche Referenz-Durchstrahlungsaufnahmen "relativ einfach" mittels Bildverarbeitungsvorgängen, wie z.B. Kreuzkorrelationsberechnungen oder Berechnungen kleinster Quadrate basierend auf den Referenz-Durchstrahlungsaufnahmen miteinander verglichen werden bzw. miteinander in Bezug gebracht werden.

Daraufhin wird die Datenaufnahme in Form der Erfassung von Mess-Durchstrahlungsaufnahmen des Untersuchungsobjekts begonnen. Beispielsweise kann die Datenaufnahme (d.h. die erste Mess-Durchstrahlungsaufnahme) an einer Mess-Winkelposition begonnen werden, die mit einer der Referenz-Winkelpositionen übereinstimmt.

Bei der Ermittlung eines ersten Teilsatzes von Mess-Durchstrahlungsaufnahmen an m Mess-Winkelpositionen (mit m < k) werden also m Mess-Durchstrahlungsaufnahmen des Untersuchungsobjekts erfasst. Falls nun die Datenerfassung an einer Referenz-Position begonnen wurde, kann die erste Mess-Durchstrahlungsaufnahme zusätzlich auch als Referenz-Durchstrahlungsaufnahme verwendet werden.

Daraufhin wird eine Referenz-Durchstrahlungsaufnahme des Untersuchungsobjekts an der weiteren Referenz-Winkelposition durchgeführt, wobei beispielsweise bei durch einen Bildverarbeitungsvorgang bzw. Bildvergleich mit einer vorhergehenden Referenz-Durchstrahlungsaufnahme Informationen über Störeinflüsse anhand dieser Referenz-Durchstrahlungsaufnahmen zwischen den beiden Referenz-Zeitpunkten, zu denen die unterschiedlichen Referenz-Durchstrahlungsaufnahmen ermittelt wurden, abgeleitet werden können. Sind nun keine Störeinflüsse feststellbar, unterscheiden sich die Referenz-Durchstrahlungsaufnahmen an denselben Winkelpositionen lediglich durch einen Rauschanteil im Bild. Ferner können nun weitere Informationen über Störeinflüsse für z.B. alle zeitlich zwischen den Referenz-Durchstrahlungsaufnahmen aufgenommenen Mess-Durchstrahlungsaufnahmen, z.B. durch eine Interpolation, abgeleitet werden. Nachdem Störeinflussinformationen bzw. entsprechende Korrekturinformationen vorliegen, kann nun unmittelbar mit der Rekonstruktion der Mess-Durchstrahlungsaufnahmen begonnen werden.

Die oben dargestellte Vorgehensweise zur Erfassung einer Abfolge unterschiedlicher Teilsätze von Mess-Durchstrahlungsaufnahmen des Untersuchungsobjekts an vorgegebenen Mess-Winkelpositionen und das wiederholte Erfassen einer Referenz-Durchstrahlungsaufnahme des Untersuchungsobjekts zu unterschiedlichen Referenz-Zeitpunkten jeweils zeitlich zwischen zwei Erfassungsvorgängen für die unterschiedlichen Teilsätze von Mess-Durchstrahlungsaufnahmen wird nun wiederholt, bis der vollständige Durchstrahlungsdatensatz mit allen erforderlichen bzw. vorgesehenen Mess-Durchstrahlungsaufnahmen, d.h. der Gesamtzahl k von Mess-Durchstrahlungsaufnahmen, erhalten wurde. Bei der erfindungsgemäßen Vorgehensweise ist zu beachten, dass die Anzahl m der aufzunehmenden Mess-Durchstrahlungsaufnahmen zwischen zwei Referenz-Durchstrahlungsaufnahmen und damit die Abtastfrequenz der Referenz-Durchstrahlungsaufnahmen über der Messdauer hinweg nicht konstant sein braucht. Beispielsweise könnte man die Anzahl m (Anzahl der Mess-Durchstrahlungsaufnahmen pro Teilsatz) mit zunehmender Messdauer vergrößern oder dynamisch zur Laufzeit anhand der Ergebnisse der Vergleiche vorhergehender Referenz-Durchstrahlungsaufnahmen anpassen. In diesem Zusammenhang wird ferner darauf hingewiesen, dass mit der Aufnahme eines weiteren Teilsatzes von Mess-Durchstrahlungsaufnahmen des Untersuchungsobjekts bereits begonnen werden kann, bevor der Ableitungsvorgang der Informationen über die Störeinflüsse der vorangegangenen Teilsätze abgeschlossen ist.

Die Anzahl der Erfassungsvorgänge für Referenz-Durchstrahlungsaufnahmen gibt ein Maß für die zeitliche Abtastung der Störeinflüsse bzw. Änderung der Abbildungsgeometrie an. Die Reihenfolge der Erfassung bzw. Ermittlung der eigentlichen Mess-Durchstrahlungsaufnahmen kann im Wesentlichen beliebig vorgenommen werden. So kann beispielsweise die Aufnahme der Mess-Durchstrahlungsaufnahmen im Fall von n Stützstellen (d.h. Referenzinformationen basierend auf den Referenz-Durchstrahlungsaufnahmen) zu n - 1 gleichen Teilen aufgeteilt werden, damit die zeitliche Abtastung der Stützstellen möglicht gleichmäßig über den Datensatz (Durchstrahlungsdatensatz bzw. Projektionsdatensatz) bzw. die Messzeit verteilt stattfindet. Eine Stützstelle (Referenzinformation) basiert nun auf einer oder einer Mehrzahl von Referenz-Durchstrahlungsaufnahmen zu einem Referenzzeitpunkt. Bei dem Verfahren zur Datenermittlung für eine möglichst Störeinfluss-reduzierte Computertomographieaufnahme eines Untersuchungsobjekts in einem CT-System stellt somit das Untersuchungsobjekt selbst das Referenzobjekt bzw. die Referenzstruktur dar.

Aus der Bildsequenz der Referenz-Durchstrahlungsaufnahmen können die relativen Positionen der Abbildungsgeometrie und insbesondere des optischen Brennflecks des Röntgengeräts zu diskreten Zeitpunkten bestimmt werden. Diese zeitdiskreten Punkte stellen die Stützstellen für die Bestimmung einer im Hinblick auf Ort- und Zeit stetigen Funktion dar, welche die relative Position des optischen Brennflecks zu jedem Zeitpunkt des Messzeitraums approximativ beschreibt. Somit lässt sich anhand dieser Funktion die relative Position des optischen Brennflecks für jede Projektion auf Grundlage einer Interpolation herleiten. Die Güte der Herleitung kann dabei durch ein (endliches) Erhöhen der zeitlichen Abtastfrequenz der Referenzprojektionen verbessert werden. Diese Störeinflussfunktion bzw. Korrekturfunktion, welche die relative Brennfleckposition zu jedem Zeitpunkt im Messzeitraum beschreibt, kann nun dem Rekonstruktionsalgorithmus als projektionsbezogene Zusatzinformation zur Verfügung gestellt werden. Somit kann der Tiefpasscharakter eines sich bewegenden Brennflecks bei der nachfolgenden Rekonstruktion einer 2D- oder 3D-CT-Aufnahme kompensiert werden. Dies führt zu einer höheren Auflösung bzw. Detailerkennbarkeit des Messsystems.

Die erfindungsgemäße Vorgehensweise zeichnet sich also dadurch aus, dass diese als Grundlage für die Detektion und Kompensation von zeitabhängigen Störeinflüssen bzw. geometrischen Abbildungsänderungen eingesetzt werden kann. Geometrische und andere Einflussfaktoren können mit diesem Verfahren zuverlässig detektiert und quantifiziert werden.

Das erfindungsgemäße Konzept ermöglicht daher eine hohe Präzision der Kompensation der Brennfleckbewegung. Neben der Winkelabhängigkeit der Bestimmbarkeit der Brennfleckposition wird auch die zeitliche Komponente einbezogen, so dass abhängig von der Abtastgeschwindigkeit auch hochfrequente bzw. sich schnell ändernde Brennfleckbewegungen detektiert und kompensiert werden können. Dies führt unmittelbar zu einer reduzierten Unschärfe, also zu einer verbesserten Bildqualität in den Rekonstruktionsdaten. Auf Basis der verbesserten Bilddaten können präzisere und robustere Analyse- und Messergebnisse entsprechend der jeweiligen Untersuchungsaufgabe erzielt werden. Diese Verbesserung der Bildqualität kann insbesondere auch ohne den Einsatz von Referenzkörpern erzielt werden. Es kann daher die maximale Bildgröße für die Abbildung des Untersuchungsobjekts genutzt werden, wodurch eine maximale Ortsauflösung und Detailerkennbarkeit realisiert werden können.

Über die Detektion und Kompensation einer Brennfleckbewegung hinaus kann mit der vorliegenden Vorgehensweise auch eine Veränderung der Vergrößerung in der Abbildung erkannt und kompensiert werden sowie unter Randbedingungen auch eine Bewegung des Objekts.

Im Folgenden wird nun anhand von Dokumenten zum Stand der Technik auf Probleme herkömmlicher CT-Aufnahmesysteme eingegangen, wobei ferner die Erkenntnisse und erfindungsgemäßen Schlussfolgerungen der Erfinder unter Berücksichtigung der der vorliegenden Erfindung zugrunde liegenden Aufgabe herausgestellt werden.

Einige Vorgehensweisen zur zeitlichen Bestimmung und Korrektur der Veränderung der Anlagengeometrie eines CT-Systems anhand von Bilddaten basieren auf den der wissenschaftlichen Veröffentlichung "A. Sasov, S. Liu, P. Salmon, Proc. Of SPIE, 7078, 70781C-1, 2008", die an SEM-Tomographien (SEM = Scanning Electron Microscope) erprobt wurden.

Ein darin beschriebenes iteratives Verfahren mittels Rück- und Vorwärts-Projektion verwendet ein zunächst unkorrigiertes, potenziell Artefakt-behaftetes Rekonstruktionsvolumen, um anhand dessen mittels Strahlsummenmethode einen zu erwartenden Durchstrahlungsdatensatz (Projektionsdatensatz) zu generieren. Dieser Datensatz wird projektionsweise mit dem aufgenommenen Durchstrahlungsdatensatz verglichen. Für jede Durchstrahlungsaufnahme wird mittels des Verfahrens der kleinsten Quadrate oder über eine Kreuzkorrelation eine geschätzte Brennfleckprojektion ermittelt. Eine erneute Rekonstruktion des gesamten Durchstrahlungsdatensatzes erfolgt dann unter Einbeziehung dieser Schätzwerte. Da nun angenommen wird, dass die Schätzwerte des ersten Schritts durch weitere Iterationen noch verbessert werden können und das Fehlermaß und somit die Artefakte im Bild weiter reduziert werden können, wird dieses Verfahren wiederholt bis ein Schwellwert für die Fehlertoleranz unterschritten wird. Das zuletzt generierte rekonstruierte Volumen entspricht dann dem endgültigen korrigierten Volumen.

Bei einem sogenannten schnellen Pre- oder Post-Scan-Vorgang wird unmittelbar vor oder nach der Messung des gesamten Durchstrahlungsdatensatzes ein weiterer schneller Scan des Objekts durchgeführt. Vorgeschlagen wird beispielsweise eine Winkelschrittweite von 30° oder 45°, was bei einer vollen Abtastung von 360° einem Datensatz von 12 bzw. 8 Durchstrahlungsaufnahmen (Projektionsbildern) entspricht. Die Aufnahme eines solchen Referenzdatensatzes am SEM soll innerhalb von zwei Minuten abgeschlossen sein. Werden nun konventionelle CT-Systeme mit Röntgenröhren als Strahlenquelle eingesetzt, ist mit einer Reduktion der notwendigen Messzeit für den Referenzdatensatz zu rechnen, da mit Röntgenröhren in der Regel ein deutlich höherer Photonenfluss erzeugt werden kann. Für die acht oder zwölf Referenzpositionen wird nun wiederum mit der Methode der kleinsten Quadrate oder mittels einer Kreuzkorrelation unter Beachtung der tatsächlich gemessenen Daten Werte für die Brennfleckposition ermittelt. Diese Werte werden relativ zu der Position des Brennflecks, an der sich derselbe vor oder nach der Messung befand, ermittelt, je nachdem, ob der schnelle Scan vor oder nach der Messung durchgeführt wurde.

Die beiden obigen Verfahren basieren auf dem Vergleich der aufgenommenen Durchstrahlungsaufnahmen mit Referenzdurchstrahlungsaufnahmen, anhand dessen ein Korrekturwert für den Ausgleich der Brennfleckbewegung ermittelt wird. Im Falle des iterativen Verfahrens ist dies für jede Durchstrahlungsaufnahme separat möglich. Für jede Durchstrahlungsaufnahme kann also ein von dem restlichen Datensatz unabhängiger Korrekturwert ermittelt werden. Jedoch ist es für dieses Verfahren erforderlich, dass eine vollständige Rekonstruktion des Datensatzes inklusiver aller Bewegungsartefakte vorliegt, bevor die Ermittlung der Korrekturwerte beginnen kann. Die Ermittlung der Korrekturwerte kann daher frühestens mit der Aufnahme der letzten Durchstrahlungsaufnahme des Datensatzes beginnen. Da es sich um ein iteratives Verfahren handelt, hängt es zudem von dem Konvergenzverhalten ab, wie schnell die Ermittlung der Parameter abgeschlossen ist und die endgültige Artefakt-reduzierte Rekonstruktion vorliegt.

Im Falle des schnellen Scans vor oder nach der Messung, d.h. nach der Ermittlung des gesamten Durchstrahlungsdatensatzes mit allen Durchstrahlungsaufnahmen, werden zusätzliche Messdaten zu den für die Rekonstruktion des Untersuchungsobjekts relevanten Durchstrahlungsdatensatzes generiert. Dieser Ansatz folge der Annahme, dass sich die Position des Brennflecks während des schnellen Scans, für den typische Messzeiten von ca. zwei Minuten angegeben werden, nicht ändert. Auch bei deutlich kürzeren Messzeiten haben Versuche jedoch gezeigt, dass diese Annahme im Allgemeinen nicht zutrifft. Eine potenzielle Brennfleckwanderung während der Aufnahme des schnellen Scans wird mit diesem Verfahren in den vollständigen Durchstrahlungsdatensatz (Projektionsdatensatz) des Untersuchungsobjekts eingerechnet und verschlechtert diesen gegebenenfalls. Zudem bringt dieses Verfahren dahin gehend ein Fehlerpotenzial ein, dass der Vergleich der Projektionen je nach Objektbeschaffenheit aus unterschiedlichen Winkeln unterschiedlich präzise Ergebnisse liefert. Es ist also nicht damit zu rechnen, dass für alle 8 oder 12 Stützstellen die Brennfleckposition gleichermaßen präzise ermittelt werden kann.

Bezüglich der vorliegenden Erfindung wurde nun erkannt, dass es präziser ist, eine Winkelposition zu wählen, in der eine Bildregistrierung oder andere Bildverarbeitungsmethoden/Verfahren zur Ermittlung der Brennfleckposition möglich sind, bei denen robust reproduzierbare Ergebnisse mit geringem Fehler zu erwarten sind, und diese Winkelpositionen dann als Referenzposition(en) zu verwenden. Dieser Ansatz wird bei Ausführungsbeispielen der vorliegenden Erfindung eingesetzt, um objektabhängige Referenzpositionen für aussagekräftige Referenz-Durchstrahlungsaufnahmen des Untersuchungsobjekts zu erhalten. Dies wird im bisherigen Stand der Technik nicht erkannt.

Ferner wird hinsichtlich der wissenschaftlichen Veröffentlichung "A. Sasov, S. Liu, P. Salmon, Proc. Of SPIE, 7078, 70781C-1, 2008" darauf hingewiesen, dass als Spezialfall des sogenannten Post-Scans häufig zu beobachten ist, dass nach der eigentlichen Messung lediglich die erste Durchstrahlungsaufnahme erneut aufgenommen und mit der ersten Durchstrahlungsaufnahme aus dem Datensatz verglichen wird. Eine detektierte Brennfleckbewegung zwischen den beiden Referenzprojektionen wird als linear angenommen und alle weiteren Projektionen mittels linearer Interpolation korrigiert. Zwar mag diese Vorgehensweise bei hohen Ortsauflösungen bereits eine Verbesserung im Vergleich zu der unkorrigierten Rekonstruktion der Daten ergeben, die Annahme einer linearen Brennfleckbewegung wird jedoch im Allgemeinen nicht der tatsächlichen Bewegung entsprechen. Insbesondere hochfrequente Brennfleckbewegungen werden durch diese vereinfachte Annahme nicht detektiert und bleiben unberücksichtigt. Somit erreicht diese Vorgehensweise zur Detektion zum Ausgleich der Brennfleckbewegung weder eine stark erhöhte Präzision der ermittelten Brennfleckpositionen noch eine verbesserte Zuordnung zu den gemessenen Projektionen, wobei insbesondere ein relativ hoher zusätzlicher Aufwand erforderlich ist, da erst nach vollständiger Datenaufnahme mit der Rekonstruktion begonnen werden kann.

Die WO 2008/141825 A3 bezieht sich auf ein Verfahren, eine Vorrichtung und eine Anordnung zur Aufnahme von Röntgenprojektionsbildern, wobei mittels einer Röntgenröhre und eines röntgensensitiven Detektors zeitlich aufeinanderfolgend mehrere Projektionen eines Untersuchungsobjekts unter unterschiedlichen Winkelstellungen aufgenommen werden, und wobei ein Referenzobjekt auf den Detektor abgebildet wird, aus der Lage des Referenzobjekts in den Abbildungen der Projektionen die Wanderung der früheren Brennfleckposition berechnet wird, und für die zweite und die darauf folgende Projektionen diese Wanderung jeweils kompensiert wird, bevor die jeweils zeitlich nachfolgenden Projektion aufgenommen wird.

Gemäß der WO 2008/141825 A3 wird eine sofortige Kompensation der Brennfleckbewegung in den Projektionen durch Methoden der Bildverarbeitung sowie zusätzlich durch eine tatsächliche mechanische Bewegung der Strahlungsquelle bzw. alternativ des Prüfobjekts und den Röntgendetektors durchgeführt. Die Kompensation der Brennfleckbewegung durch Änderung der Abbildungsgeometrie, z.B. durch eine Bewegung der Strahlungsquelle, des Untersuchungsobjekts und/oder des Detektors ist jedoch definitionsgemäß mit einer zeitlichen Latenz verbunden und wird für hochauflösende Bildgebungsverfahren als nicht ausreichend präzise eingeschätzt. Die Brennfleckbewegung wird zudem mithilfe von ortsunveränderlichen Referenzobjekten, z.B. Stahlkugeln, Blenden, detektiert, die in diesem Fall definitionsgemäß nicht das Untersuchungsobjekt sind, da dieses nicht ortsunveränderlich bleibt. Durch dieses Verfahren ist zwar eine zeitnahe Kompensation der Brennfleckbewegung in den Projektionen möglich, wobei aber durch die zusätzliche Abbildung von ortsunveränderlichen Referenzobjekten ein Teil der aktiven Fläche des Röntgendetektors von Objekten abgeschattet wird, die nicht Teil des zu untersuchenden Objekts sind und somit keine für die Prüf- oder Messaufgabe relevante Information tragen. Der prüfbare Objektbereich wird somit eingeschränkt.

Bevorzugte Ausführungsbeispiele der vorliegenden Erfindung werden nachfolgend Bezug nehmend auf die beiliegenden Zeichnungen näher erläutert. Es zeigen:
- Fig. 1: eine Prinzipdarstellung einer erfindungsgemäßen CT-Anlage gemäß einem Ausführungsbeispiel der Erfindung;
- Fig. 2: ein Flussdiagramm eines Verfahrens zur Datenermittlung für eine Computertomographieaufnahme eines Untersuchungsobjekts gemäß einem Ausführungsbeispiel der vorliegenden Erfindung; und
- Fig. 3: ein weiteres Flussdiagramm eines Verfahrens zur Datenermittlung für eine Computertomographieaufnahme eines Untersuchungsobjekts gemäß einem weiteren Ausführungsbeispiel der vorliegenden Erfindung.

Bevor nachfolgend Ausführungsbeispiele der vorliegenden Erfindung im Detail anhand der Zeichnungen näher erläutert werden, wird darauf hingewiesen, dass identische, funktionsgleiche oder gleichwirkende Elemente, Objekte und/oder Strukturen in den unterschiedlichen Figuren mit den gleichen Bezugszeichen versehen sind, so dass die in unterschiedlichen Ausführungsbeispielen dargestellte Beschreibung dieser Elemente untereinander austauschbar ist bzw. aufeinander angewendet werden kann.

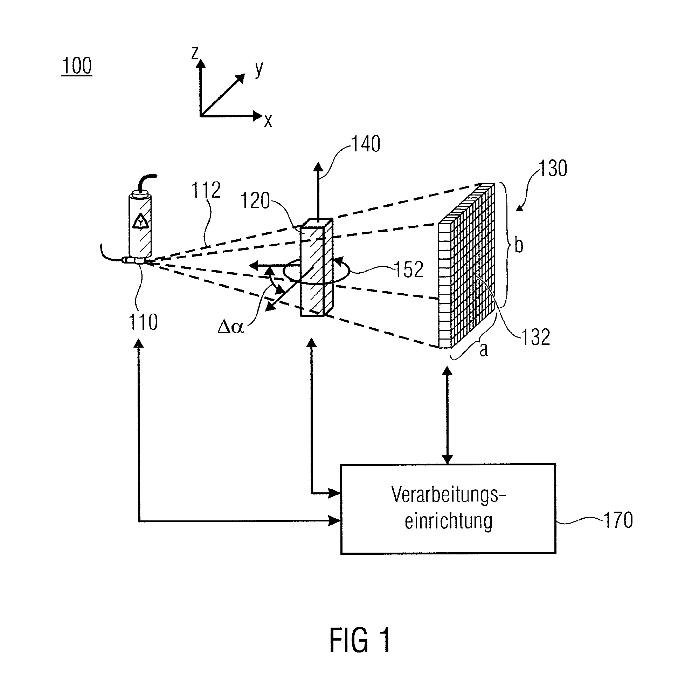

Fig. 1 zeigt eine Darstellung eines CT-Systems 100 gemäß einem Ausführungsbeispiel der vorliegenden Erfindung zur Datenermittlung für eine (möglichst Störeinfluss-freie bzw. Störeinfluss-reduzierte) Computertomographieaufnahme eines Untersuchungsobjekts 120 (Prüfkörpers). Das CT-System 100 weist beispielsweise eine Röntgenröhre 110 und einen für Röntgenstrahlung 112 empfindlichen (flächigen) Detektor 130, z.B. in Form eines Flachbilddetektors bzw. flächigen Röntgendetektors, auf. Die von der Röntgenstrahlungsquelle 110 austretende Röntgenstrahlung 112 durchstrahlt bzw. durchdringt das Untersuchungsobjekt 120 und trifft auf den röntgenempfindlichen Detektor 130.

Nach Transmission bzw. Durchgang der Röntgenstrahlung 112 durch das Untersuchungsobjekt 120 entsteht eine einzelne Mess-Durchstrahlungsaufnahme. Die Mess-Durchstrahlungsaufnahme beinhaltet die Bildinformationen über die Linienintegrale über die Schwächungskoeffizienten beim Durchgang der Röntgenstrahlung 112 durch das Untersuchungsobjekt 120.

Ferner ist dem CT-System 100 eine Verarbeitungs- und Steuerungseinrichtung 170 zugeordnet, die zur Ansteuerung des CT-Systems 100 einschließlich der Röntgenstrahlungsquelle 110 und des röntgenempfindlichen Detektors 130, zur Ansteuerung der Bewegung des Untersuchungsobjekts 120 und ferner zur Auswertung der erfassten Durchstrahlungsbilder (Mess- und auch Referenz-Durchstrahlungsbilder) dient. In Fig. 1 weist die Röntgendurchstrahlungsaufnahme eine Projektion des dreidimensionalen Volumens des Untersuchungsobjekts 120 auf, wobei die Durchstrahlungsaufnahme bzw. Projektion dadurch entsteht, dass die von der Röntgenstrahlungsquelle 110 austretenden Röntgenstrahlen 112 nach Durchgang durch den Prüfkörper 120 auf die zweidimensionale Oberfläche 132 des röntgenempfindlichen Detektors 130 abgebildet werden. Der röntgenempfindliche Detektor 130 ist beispielsweise als ein Festkörperdetektor ausgebildet und kann als Zeilendetektor, z.B. bei einer Flächenstrahl-CT, oder als Mehrzeilen- bzw. Flächendetektor, z.B. bei einer 3D-Konusstrahl-CT, ausgebildet sein.

Bei der in Fig. 1 gezeigten beispielhaften Anordnung weist die CT-Anlage 100 eine 3D-Konusstrahl-CT auf, wobei die Röntgenstrahlungsquelle 110 im Fokus bzw. Brennfleck als näherungsweise punktförmig angesehen werden kann, und wobei der röntgenempfindliche Detektor 130 als ein Mehrzeilendetektor mit einer zweidimensionalen Oberfläche von beispielsweise a x b Pixel ausgebildet ist. Bei der Datenermittlung, beispielsweise für eine 3D-Abbildung des Untersuchungsobjekts 120 mittels Computertomographie, wird das Untersuchungsobjekt 120 mit einer beispielsweise gleichmäßigen Winkelschrittweite Δα um eine Rotationsachse 140 gedreht, während der Rotation des Prüfkörpers 120 ein Winkelbereich 152 (beispielsweise ein vollständiger Winkelbereich von 360°) in einer Rotationsebene senkrecht zur Rotationsachse 140 überschritten wird, um eine Folge von Durchstrahlungsaufnahmen an den zugeordneten Winkelpositionen zu erhalten.

Bei der industriellen CT wird beispielsweise der Projektionsdatensatz, der als Grundlage für die Rekonstruktion von Tiefeninformationen des Untersuchungsobjekts 120 dient, üblicherweise innerhalb einer vollständigen Umdrehung des Untersuchungsobjekts 120 oder der Röntgenkomponenten im Fall eines Gantry-System um seine Rotationsachse in kleinen Winkelschritten akquiriert. Dabei ist das CT-System aus Röntgenstrahlungsquelle 110 und Röntgendetektor 130 relativ zu dem Untersuchungsobjekt 120 rotierbar angeordnet. Abhängig von der Winkelzahl wird beispielsweise der Vollkreis in äquidistanten Winkelpositionen Δα aufgeteilt, die der Reihe nach angefahren werden.

Alternativ kann die Aufnahme der Durchstrahlungsaufnahmen auch dadurch erfolgen, dass das Untersuchungsobjekt 120 um jegliche andere Rotationsachse, ausgenommen der Rotationsachse parallel zu x-Achse (beispielsweise um eine Rotationsachse parallel zur y-Achse in der x-, z-Ebene) gedreht wird, um einen vollständigen Satz von Mess-Durchstrahlungsaufnahmen zu erhalten. Jede Durchstrahlungsaufnahme weist die Bildinformation in Form einer 2D-Matrix von Durchstrahlungswerten, die typischerweise als Intensitätswerte vorliegen, auf. Mittels einer Rechner-basierten Auswertung, die beispielsweise auf der Basis einer mathematischen Transformation (z.B. Radon-Transformation) durchgeführt werden kann, wird aus einer Vielzahl der Mess-Durchstrahlungsaufnahmen (aus dem Durchstrahlungsdatensatz bzw. Projektionsdatensatz) ein 3D-Bild bzw. 3D-Volumen rekonstruiert, wobei jedem Volumenelement bzw. Voxel des 3D-Bildes ein Schwächungskoeffizient bzw. Absorptionsgrad zugeordnet wird.

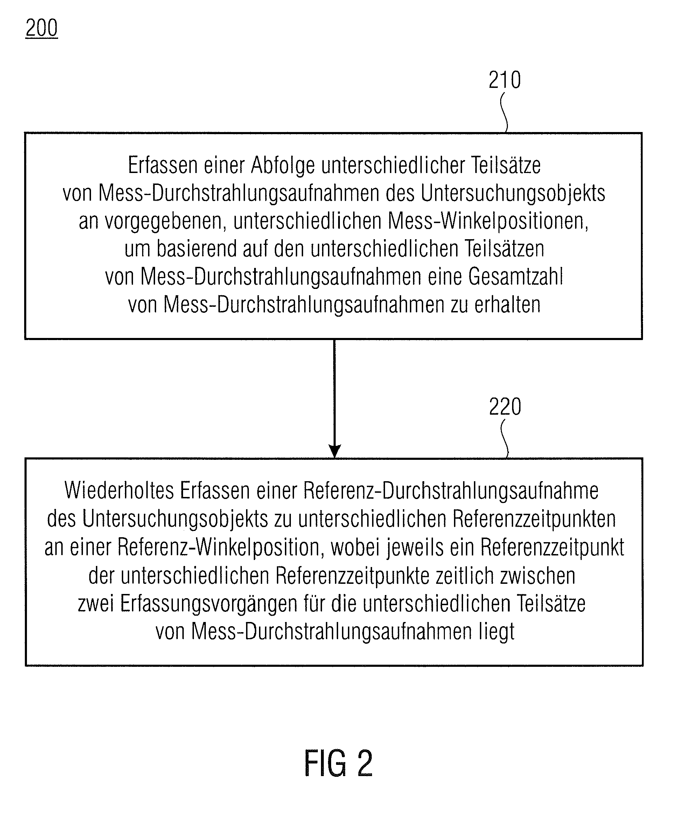

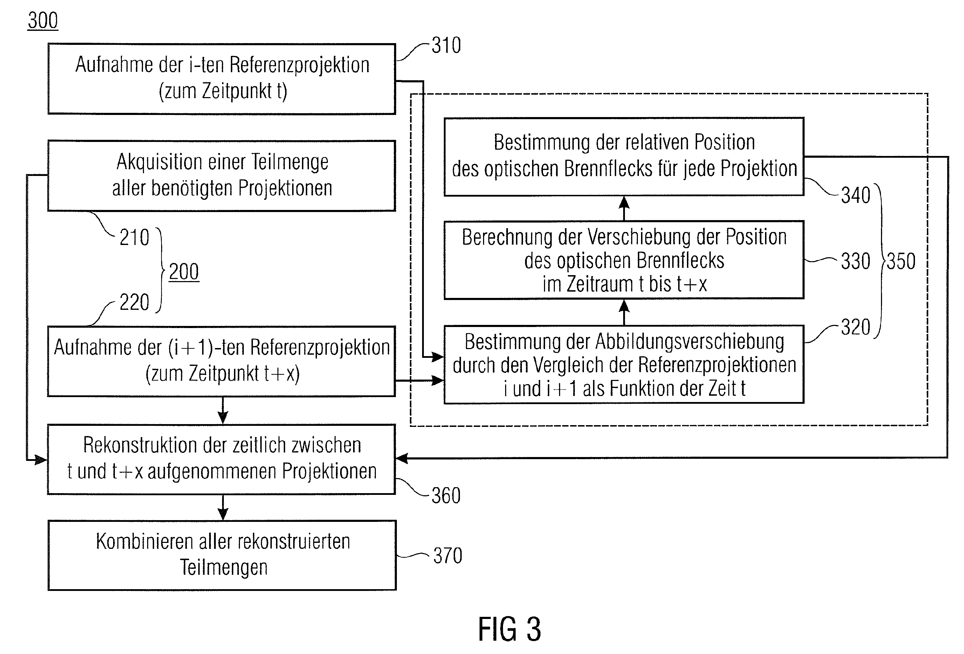

Im Folgenden wird nun anhand der in Fig. 1 dargestellten Komponenten des CT-Systems 100 und anhand der in Fig. 2 und 3 dargestellten Verfahrenschritte das erfindungsgemäße Konzept, d.h. die Funktionsweise der Komponenten des CT-Systems 100 und der Verfahrensablauf, zur Einsatz-synchronen Detektion und Kompensation von zeitabhängigen Störeinflüssen auf Mess-Durchstrahlungsaufnahmen an Computertomographiesystemen beschrieben.

Insbesondere wird die Funktionsweise der Verarbeitungs- und Steuerungseinrichtung 170 und ferner der zugehörige Verfahrensablauf zur Datenermittlung für eine Computertomographieaufnahme des Untersuchungsobjekts bzw. Prüfkörpers 120 in einer CT-Anordnung mit der Röntgenstrahlungsquelle 110 und dem Röntgendetektor 130 erläutert. Dabei sind die CT-Anordnung und das Untersuchungsobjekt 120 relativ zueinander rotierbar angeordnet. Wenn bei der nachfolgenden Beschreibung angegeben wird, dass unterschiedliche Winkelpositionen des Untersuchungsobjekts 120 hinsichtlich der Röntgenstrahlungsquelle 110 und des Detektors 130 (bzw. einer durch dieselben gebildeten Durchstrahlungsebene) eingestellt werden, sollte deutlich sein, dass dazu beispielsweise geeignete Stelleinrichtungen (Stellmotoren) verwendet werden können, um exakt die gewünschten Winkelpositionen (Mess- und/oder Referenz-Winkelpositionen) für die jeweiligen Durchstrahlungsaufnahmen zu erhalten. Diese Stelleinrichtungen werden von der Verarbeitungs- und Steuerungseinrichtung 170 angesteuert, wobei bei einer nachfolgenden Beschreibung zur Vereinfachung davon gesprochen wird, dass die Verarbeitungs- und Steuerungseinrichtung 170 die jeweiligen Winkelpositionen einstellt.

Darüber hinaus wird darauf hingewiesen, dass bei der nachfolgenden Beschreibung angegeben wird, dass unterschiedliche Durchstrahlungsaufnahmen (z.B. Mess- und/oder Referenz-Durchstrahlungsaufnahmen) erfasst werden. Diese Erfassung erfolgt im Zusammenspiel mit dem Röntgendetektor 130, der dann entsprechende Detektionsdaten an die Verarbeitungs- und Steuerungseinrichtung 170 übermittelt. Die Detektionsdaten enthalten dabei die Bilddaten der jeweiligen Durchstrahlungsaufnahmen, die dann beispielsweise noch in der Verarbeitungs- und Steuerungseinrichtung 170 aufbereitet und weiterverarbeitet werden können. Die in Fig. 1 dargestellten Doppelpfeile ausgehend von der Verarbeitungs- und Steuerungseinrichtung 170 sollen angeben, dass jeweils auch eine bidirektionale Datenkommunikation zwischen den unterschiedlichen Elementen möglich ist.

Bei der industriellen Computertomographie wird ein Durchstrahlungsdatensatz (Projektionsdatensatz), der als Grundlage für die Rekonstruktion von Tiefeninformationen des Untersuchungsobjekts 120 dient, üblicherweise innerhalb einer vollständigen Umdrehung (360°) des Objekts oder der Röntgenkomponenten im Fall eines Gantry-Systems um dessen Rotationsachse in kleinen Winkelschritten, z.B. Δα ≤ 1° erfasst. Abhängig von der Winkelzahl wird der Vollkreis in äquidistante Winkelpositionen Δα geteilt, die der Reihe nach angefahren werden.

Gemäß Ausführungsbeispielen der vorliegenden Erfindung ist die Verarbeitungs- und Steuerungseinrichtung 170 mit der Computertomographieanordnung 110, 130 gekoppelt und ferner ausgebildet, um eine Abfolge unterschiedlicher Teilsätze von Mess-Durchstrahlungsaufnahmen des Untersuchungsobjekts 120 an vorgegebenen Winkelpositionen zu erfassen, um basierend auf den unterschiedlichen Teilsätzen von Mess-Durchstrahlungsaufnahmen eine Gesamtzahl von Mess-Durchstrahlungsaufnahmen zu erhalten, und um eine Referenz-Durchstrahlungsaufnahme des Untersuchungsobjekts zu unterschiedlichen Referenzzeitpunkten an einer Referenz-Winkelposition wiederholt zu erfassen, wobei jeweils einer der unterschiedlichen Referenzzeitpunkte zeitlich zwischen zwei Erfassungsvorgängen für die unterschiedlichen Teilsätze von Mess-Durchstrahlungsaufnahmen liegt.

Gemäß Ausführungsbeispielen der vorliegenden Erfindung besteht das Verfahren 200 zur Datenermittlung für eine (Störeinfluss-reduzierte) Computertomographieaufnahme eines Untersuchungsobjekts 120 in dem CT-System 100 nun darin, zunächst eine Abfolge unterschiedlicher Teilsätze bzw. Teilmengen von Mess-Durchstrahlungsaufnahmen des Untersuchungsobjekts 120 an vorgegebenen, unterschiedlichen Mess-Winkelpositionen Δα zu erfassen, um basierend auf einer Kombination der unterschiedlichen Teilsätze von Mess-Durchstrahlungsaufnahmen die Gesamtzahl von Mess-Durchstrahlungsaufnahmen zu erhalten. Ferner wird eine Referenz-Durchstrahlungsaufnahme des Untersuchungsobjekts 120 zu unterschiedlichen Referenz-Zeitpunkten t + ix (mit i = 0, 1, 2, 3.... n) an zumindest einer Referenz-Winkelposition β wiederholt erfasst (Schritt 220 von Fig. 2), wobei jeweils ein Referenz-Zeitpunkt der unterschiedlichen Referenz-Zeitpunkte zeitlich zwischen zwei Erfassungsvorgängen für die unterschiedlichen Teilsätze der Mess-Durchstrahlungsaufnahmen liegt.

Die Referenz-Durchstrahlungsaufnahmen zur Bestimmung der aus der Brennfleckbewegung resultierenden Abbildungsverschiebungen werden gemäß Ausführungsbeispielen durch ein mehrfaches Anfahren derselben Aufnahmegeometrie erzeugt, d.h. zu unterschiedlichen Referenzzeitpunkten wird jeweils dieselbe Aufnahmegeometrie mit einer oder mehreren Referenz-Durchstrahlungsaufnahmen erzeugt. Dabei kann es auch erforderlich sein, dass mehrere Achsen (z.B. die Rotationsachse 140 und ggf. weiteren Achsen des Untersuchungsobjekts 120) verfahren werden, um die Referenzpositionen anzufahren.

Somit werden die Referenz-Durchstrahlungsaufnahmen, die zur Beurteilung eines etwaigen Störeinflusses auf die Mess-Durchstrahlungsaufnahmen dienen, jeweils zwischen der Erfassung unterschiedlicher Teilsätze der Mess-Durchstrahlungsaufnahmen ermittelt.

Dadurch können Störeinflüsse durch mehrmaliges Erfassen bzw. Akquirieren der gleichen Durchstrahlungsdaten (Projektionsdaten) in Form der Referenz-Durchstrahlungsaufnahmen (Referenzprojektionen) in den eigentlichen Messablauf zur Erfassung der Mess-Durchstrahlungsaufnahmen integriert werden. Während der Erfassung des Durchstrahlungsdatensatzes des Untersuchungsobjekts 120 werden also eine oder mehrere zuvor definierte Referenz-Winkelpositionen von der Rotationsachse 140 und ggf. weiteren Achsen des Untersuchungsobjekts 120 angefahren, um dort Durchstrahlungsaufnahmen des Untersuchungsobjekts 120 zu erfassen, welche als Referenz (Referenzinformationen) zur Bestimmung von Störeinflüssen auf die Erfassung der Mess-Durchstrahlungsaufnahmen herangezogen werden.

Bei dem Schritt 220 des Erfassens einer Referenz-Durchstrahlungsaufnahme zu einem Referenzzeitpunkt können auch jeweils eine Mehrzahl von Referenz-Durchstrahlungsaufnahmen des Untersuchungsobjekts 120 an unterschiedlichen, vorgegebenen Referenz-Winkelpositionen βₙ (z.B. von der Rotationsachse 140 und ggf. weiteren Achsen des Untersuchungsobjekts 120) erfasst werden, um basierend auf einer Mehrzahl von Referenz-Durchstrahlungsaufnahmen die Referenzinformationen zur Störeinfluss-Reduktion an den Mess-Durchstrahlungsaufnahmen zu ermitteln.

Gemäß Ausführungsbeispielen kann die Abfolge unterschiedlicher Teilsätze von Mess-Durchstrahlungsaufnahmen beispielsweise auf unterschiedliche Weisen aufgenommen werden. So wird beispielsweise zunächst zur Erfassung für die unterschiedlichen Teilsätze der Mess-Durchstrahlungsaufnahmen eine (relativ) große Schrittweite von > 1° (z.B. einem ganzzahligen Vielfachen von 1°, z.B. 10°, 15° etc.) angenommen, um einen gesamten Umlauf zur Erfassung eines ersten Teilsatzes von Mess-Durchstrahlungsaufnahmen an den vorgegebenen Mess-Winkelpositionen zu erhalten. Als ein Vollumlauf wird beispielsweise eine resultierende Gesamtdrehung von 0 bis 360° oder zwei gegenläufige Halbumläufe mit 0° ± 180° von einem Ausgangspunkt bei 0° aus angesehen. Gleichermaßen ist es möglich, dass auch nur ein Teilumlauf zu Ermittlung eines Teilsatzes der Mess-Durchstrahlungsaufnahmen vorgenommen wird. Ein Teilumlauf kann dabei von einem Ausgangspunkt bei 0° bis einem ganzzahligen Teilerwert, d.h. 360°/b erfolgen. Der Wert b gibt dabei die Anzahl der Teilsätze an.

Nach der Ermittlung eines ersten Teilsatzes der Mess-Durchstrahlungsaufnahmen wird nun eine Referenz-Durchstrahlungsaufnahme an der Referenz-Winkelposition zu einem ersten Referenzzeitpunkt erstellt. Die Referenz-Durchstrahlungsaufnahme kann nun an der ersten Referenz-Winkelposition vorgenommen werden. Gleichermaßen können mehrere Referenz-Durchstrahlungsaufnahmen an unterschiedlichen, vorgegebenen Referenz-Winkelpositionen erfasst werden, um die Referenzinformationen zu ermitteln.

Als Referenzzeitpunkt, d.h. der Zeitpunkt der jeweiligen Referenzaufnahme, wird nun bei dem Ermitteln einer einzigen Referenz-Durchstrahlungsaufnahme pro Referenzzeitpunkt beispielsweise der Zeitpunkt des Erfassens genau dieser Referenz-Durchstrahlungsaufnahme angenommen. Bei dem Erfassen einer Mehrzahl von Referenz-Durchstrahlungsaufnahmen an unterschiedlichen, vorgegebenen Referenz-Winkelpositionen (pro Referenzzeitpunkt bzw. Referenzintervall) ist nun der Referenzzeitpunkt beispielsweise der Zeitpunkt der ersten Referenz-Durchstrahlungsaufnahme (der Mehrzahl von Durchstrahlungsaufnahmen), der Zeitpunkt der letzten Referenz-Durchstrahlungsaufnahme (der Mehrzahl von Durchstrahlungsaufnahmen) oder ein vordefinierter Zwischenzeitpunkt, z.B. in der Mitte zwischen der ersten Referenz-Durchstrahlungsaufnahme und der letzten Referenz-Durchstrahlungsaufnahme (der Mehrzahl von Durchstrahlungsaufnahmen). Als Referenzzeitpunkt ist aber auch jeder andere definierte Zeitpunkt während des Zeitintervalls für die Aufnahme der Referenz-Durchstrahlungsaufnahmen wählbar bzw. definierbar, der einer der Referenz-Durchstrahlungsaufnahmen eines Satzes von Referenz-Durchstrahlungsaufnahmen zugeordnet ist.

Nach Abschluss des Erfassens einer oder mehrerer Referenz-Durchstrahlungsaufnahmen wird nun ein weiterer vollständiger Umlauf oder teilweiser Umlauf zum Erfassen eines weiteren Teilsatzes von Mess-Durchstrahlungsaufnahmen bei weiteren unterschiedlichen Mess-Winkelpositionen durchgeführt. Die weiteren unterschiedlichen Mess-Winkelpositionen können nun zu dem ersten Voll- und/oder Teilumlauf um einen vorgegebenen Winkelwert versetzt sein oder können eine unterschiedliche Winkelschrittweite zu den ersten bzw. vorangegangenen Mess-Winkelpositionen aufweisen, um den weiteren Teilsatz von Mess-Durchstrahlungsaufnahmen zu erhalten. Die jeweiligen Mess-Winkelpositionen können hinsichtlich einer Optimierung der zu verarbeitenden Datenmenge eingestellt werden, um möglichst wenige redundante Mess-Durchstrahlungsaufnahmen bzw. Durchstrahlungsdaten zu erhalten. Nach Abschluss der Erfassung eines weiteren Teilsatzes von Mess-Durchstrahlungsaufnahmen wird nun wiederum an dem nächsten Referenzzeitpunkt eine oder eine Mehrzahl von Referenz-Durchstrahlungsaufnahmen an der Referenz-Winkelposition bzw. an den mehreren Referenz-Winkelpositionen durchgeführt (entsprechend zu der obigen Vorgehensweise).

Gemäß Ausführungsbeispielen der vorliegenden Erfindung wird also zunächst ein erster Teilsatz von Mess-Durchstrahlungsaufnahmen über einen Teil- oder Voll-Messumlauf mit einer nächsten Winkelschrittweite erfasst, wobei die erste Winkelschrittweite zwischen 5° und 30° (oder zwischen 10° und 20°) liegt. Daraufhin wird eine Referenz-Durchstrahlungsaufnahme des Untersuchungsobjekts an einer Referenz-Winkelposition zu einem ersten Referenzzeitpunkt durchgeführt. Beispielsweise ist es auch möglich, die erste Referenz-Durchstrahlungsaufnahme vor dem Erfassen eines ersten Teilsatzes von Mess-Durchstrahlungsaufnahmen durchzuführen. Ferner ist es möglich, dass bei dem Erfassen des ersten Teilsatzes von Mess-Durchstrahlungsaufnahmen eine bzw. die erste Mess-Durchstrahlungsaufnahme auch als die erste Referenz-Durchstrahlungsaufnahme erfasst wird.

Nach dem Erfassen einer Referenz-Durchstrahlungsaufnahme des Untersuchungsobjekts wird nun ein weiterer Teilsatz von Mess-Durchstrahlungsaufnahmen mit einer weiteren Mess-Winkelschrittweite, die zu der vorangehenden Schrittweite versetzt oder unterschiedlich ist, erfasst, wobei die weitere Mess-Winkelschrittweite zwischen 5° und 30° (oder zwischen 10° und 20°) liegt. Daraufhin wird eine weitere bzw. eine weitere Mehrzahl von Referenz-Durchstrahlungsaufnahmen an der Referenz-Winkelposition bzw. der Mehrzahl von Referenz-Winkelpositionen erfasst, um eine weitere Referenzinformation zu dem weiteren Referenz-Erfassungszeitpunkt zu erhalten. Die obigen Schritte des Erfassens eines weiteren Teilsatzes von Mess-Durchstrahlungsaufnahmen und von weiteren Referenz-Durchstrahlungsaufnahmen werden nun wiederholt durchgeführt, bis die Gesamtzahl von Mess-Durchstrahlungsaufnahmen bzw. der vollständige Durchstrahlungsdatensatz (Projektionsdatensatz) und die erforderlichen Referenzinformationen vorliegen.

Bezüglich der Ermittlung der Referenz-Winkelposition bzw. der Mehrzahl von Referenz-Winkelpositionen (für die Erfassung der Referenz-Durchstrahlungsaufnahmen des Untersuchungsobjekts zu den Referenzzeitpunkten) wird darauf hingewiesen, dass diese basierend auf einer geometrischen Form oder einem Aspektverhältnis des Untersuchungsobjekts vorab ermittelt werden, um möglichst aussagekräftige Referenzinformationen von dem Untersuchungsobjekt an den Referenz-Winkelpositionen zu erhalten. Dabei werden die Referenz-Winkelpositionen möglichst gewählt, um hervorgehobene, klare Außenstrukturen einen möglichst hohen resultierenden Bildkontrast (in den Referenz-Durchstrahlungsaufnahmen), möglichst kurze resultierende Durchstrahlungslängen und/oder identifizierbare Innenstrukturen aus den Referenz-Durchstrahlungsaufnahmen zu erhalten, um möglichst exakte Informationen hinsichtlich des Störeinflusses auf die Mess-Durchstrahlungsaufnahmen zu den jeweiligen Referenzzeitpunkten ableiten zu können.

Basierend auf der jeweiligen Referenz-Durchstrahlungsaufnahmen bzw. der Mehrzahl von Referenz-Durchstrahlungsaufnahmen zu dem jeweiligen Referenzzeitpunkt können nun von der Verarbeitungs- und Steuerungseinrichtung 170 Referenzinformationen basierend auf den Referenz-Durchstrahlungsaufnahmen ermittelt werden, wobei die ReferenzInformationen den Störeinfluss auf die Mess-Durchstrahlungsaufnahme jeweils zumindest zu den unterschiedlichen Referenzzeitpunkten (zumindest näherungsweise) angeben. Dies ist mit Schritt 350 in Fig. 3 dargestellt. Daraufhin kann die CT-Aufnahme basierend auf dem Durchstrahlungsdatensatz (als Kombination der unterschiedlichen Teilsätze von Mess-Durchstrahlungsaufnahmen des Untersuchungsobjekts) und den ReferenzInformationen rekonstruiert werden, um eine möglichst Störeinfluss-kompensierte CT-Aufnahme des Untersuchungsobjekts 120 zu erhalten, wie dies in den Schritten 360 und 370 in Fig. 3 dargestellt ist.

Bezüglich der unterschiedlichen Referenz-Durchstrahlungsaufnahmen zu unterschiedlichen Referenzzeitpunkten ist anzumerken, dass ohne eine geometrische Veränderung der Abbildung oder sonstige Störeinflüsse bis auf ein Bildrauschen im Wesentlichen identische Referenz-Durchstrahlungsaufnahmen z.B. zwischen aufeinanderfolgenden Referenzzeitpunkten oder einer Abfolge von mehreren Referenzzeitpunkten entstehen. Andererseits können Änderungen in der Abbildungsgeometrie und sonstige Störeinflüsse auf die Mess-Durchstrahlungsaufnahmen unter Verwendung von Bildverarbeitungsvorgängen, z.B. unter Verwendung einer Kreuzkorrelationsberechnung oder einer Berechnung kleinster Quadrate basierend auf den Referenz-Durchstrahlungsaufnahmen oder basierend auf beliebigen anderen denkbaren Bildbearbeitungsmethoden detektiert werden. Die aktuell aufgenommene Referenz-Durchstrahlungsaufnahme wird dafür in Bezug zu der zuletzt aufgenommenen, der zuerst aufgenommenen oder einer beliebigen anderen Referenz-Durchstrahlungsaufnahme an derselben Achsenposition (Referenz-Winkelposition) gesetzt. Dies ist gleichermaßen auf die Ermittlung einer Mehrzahl von Referenz-Durchstrahlungsaufnahmen anwendbar.

Abbildungsänderungen, die über ein Bildrauschen hinausgehen und auf Änderungen in der Abbildungsgeometrie oder auf andere Störeinflüsse auf die erfassten Mess-Durchstrahlungsaufnahmen zurückzuführen sind, können so "zeitabhängig" detektiert werden. Abhängig von der Anzahl der aufgenommenen Referenz-Durchstrahlungsaufnahmen (Stützstellen bzw. Referenzprojektionen) kann eine hohe zeitliche Abtastung und damit einhergehend die Detektion zeitlich hochfrequenter (schnell wechselnder) Störeinflüsse erreicht werden.

Im Folgenden wird nun anhand von Fig. 3 ein weiteres Ausführungsbeispiel eines Verfahrens zur Datenermittlung für eine Störeinfluss-reduzierte Computertomographieaufnahme eines Untersuchungsobjekts dargestellt. Dabei wird angenommen, dass die Erfassung des Durchstrahlungsdatensatzes auf k Durchstrahlungsaufnahmen, d.h. auf einer Gesamtzahl von k Winkelschritten, basiert.

Zunächst werden eine oder mehrere aussagekräftige Referenz-Winkelpositionen abhängig von dem jeweiligen Untersuchungsobjekt definiert bzw. gewählt. Diese definierten Referenz-Winkelpositionen sollen ermöglichen, dass aus den unterschiedlichen Referenz-Durchstrahlungsaufnahmen bzw. den daraus resultierenden Referenzinformationen zu unterschiedlichen Referenzzeitpunkten erstellt werden können, die möglichst einfach und definiert zueinander in Bezug gesetzt werden können, um einen effektiven Vergleich zugeordneter Referenz-Durchstrahlungsaufnahmen mittels Bildverarbeitungsmethoden zu vereinfachen bzw. effektiver gestalten zu können, und um somit umso genauer etwaige geometrische Veränderungen der Abbildung und sonstige Störeinflüsse ermitteln zu können.

Daraufhin wird mit der Datenaufnahme in Form der Erfassung von Mess- und Durchstrahlungsaufnahmen und Referenz-Durchstrahlungsaufnahmen des Untersuchungsobjekts 120 begonnen. Beispielsweise kann die Datenaufnahme an einer Referenzposition begonnen werden (Schritt 310), so dass beispielsweise die erste ermittelte Durchstrahlungsaufnahme (auch) als Referenz-Durchstrahlungsaufnahme verwendet werden kann.

Bei der Ermittlung eines (ersten) Teilsatzes von Mess-Durchstrahlungsaufnahmen an m Mess-Winkelpositionen mit m < k werden also m Mess-Durchstrahlungsaufnahmen des Untersuchungsobjekts erfasst (Schritt 210 von Fig. 3). Falls nun die erste Mess-Winkelposition mit einer (ersten) Referenz-Winkelposition übereinstimmt, kann nun die erste Mess-Durchstrahlungsaufnahme zusätzlich auch als Referenz-Durchstrahlungsaufnahme verwendet werden.

Daraufhin wird nun eine (weitere) Referenz-Durchstrahlungsaufnahme des Untersuchungsobjekts 120 an der Referenz-Winkelposition durchgeführt. Es wird also die (i + 1)-te Referenz-Durchstrahlungsaufnahme zum Zeitpunkt t + ix aufgenommen (Schritt 220 von Fig. 3).

Dabei können nun beispielsweise durch einen Bildverarbeitungsvorgang bzw. Bildvergleich mit einer vorhergehenden Referenz-Durchstrahlungsaufnahme Informationen (Referenzinformationen) über Störeinflüsse anhand dieser Referenz-Durchstrahlungsaufnahmen zwischen den beiden Referenzzeitpunkten abgeleitet werden, zu denen die unterschiedlichen Referenz-Durchstrahlungsaufnahmen ermittelt wurden. Sind nun beispielsweise keine Störeinflüsse aufgetreten und damit feststellbar, so unterscheiden sich die Referenz-Durchstrahlungsaufnahmen an denselben Winkelpositionen zu unterschiedlichen (z.B. aufeinanderfolgenden) Referenzzeitpunkten lediglich durch einen Rauschanteil im Bild. Sind dagegen Störeinflüsse in dem Untersuchungsintervall vorhanden, können nun weitere Referenzinformationen über die Störeinflüsse, wie z.B. alle zeitlich zwischen den Referenz-Durchstrahlungsaufnahmen aufgenommenen Mess-Durchstrahlungsaufnahmen, z.B. durch eine Interpolation, abgeleitet werden (siehe Schritt 350 von Fig. 3). Nachdem nun die Störeinflussinformationen bzw. entsprechende Korrekturinformationen vorliegen, kann nun unmittelbar mit der Rekonstruktion der Mess-Durchstrahlungsaufnahmen, die zeitlich zwischen t und t + x aufgenommen wurden, begonnen werden (siehe Schritt 360 von Fig. 3).

Das Ermitteln der Referenzinformationen (Schritt 350 von Fig. 3) kann nun beispielsweise in folgende Schritte bzw. Teilschritte aufgeteilt werden. Ausgehend von den zu unterschiedlichen Referenzzeitpunkten (z.B. zwischen den Referenzzeitpunkten t und t + x (mit x als Zeitintervall zwischen zwei Referenzzeitpunkten) erhaltenen Referenz-Durchstrahlungsaufnahmen ((i-te) und (i + 1)-te Referenz-Durchstrahlungsaufnahme) kann nun zunächst die Abbildungsverschiebung bzw. Geometrieänderung durch den Vergleich der Referenz-Durchstrahlungsaufnahmen i und i + 1 als Funktion der Zeit t bestimmt werden (Schritt 320 von Fig. 3). Daraufhin kann die Verschiebung der Position des optischen Brennflecks zu den Zeitpunkten t und t + x bzw. für den Zeitraum zwischen t und t + x (mittels einer mathematischen Interpolation) berechnet werden (Schritt 330 in Fig. 3). Daraufhin kann die relative Position des optischen Brennflecks für jede Mess-Durchstrahlungsaufnahme bestimmt werden (Schritt 340 von Fig. 3), und bei der Rekonstruktion der zeitlich zwischen t und t + x aufgenommenen Mess-Durchstrahlungsaufnahmen berücksichtigt werden (Schritt 360 in Fig. 3), um Störeinfluss-reduzierte bzw. Störeinfluss-kompensierte Teilsätze von Mess-Durchstrahlungsaufnahmen zu erhalten. Eine Kombination aller rekonstruierten Teilsätze von Mess-Durchstrahlungsaufnahmen führt zu der rekonstruierten Computertomographieaufnahme (Schritt 370 von Fg. 3).

Die oben dargestellte Vorgehensweise zur Erfassung einer Abfolge unterschiedlicher Teilsätze von Mess-Durchstrahlungsaufnahmen des Untersuchungsobjekts 120 an vorgegebenen Mess-Winkelpositionen und das wiederholte Erfassen einer Referenz-Durchstrahlungsaufnahme (bzw. einer Mehrzahl von Referenz-Durchstrahlungsaufnahmen) des Untersuchungsobjekts 120 zu unterschiedlichen Referenzzeitpunkten jeweils zeitlich zwischen zwei Erfassungsvorgängen für unterschiedliche Teilsätze der Mess-Durchstrahlungsaufnahmen wird nun solange wiederholt, bis der vollständige Durchstrahlungsdatensatz mit allen erforderlichen bzw. vorgesehenen k Mess-Durchstrahlungsaufnahmen erhalten wurde (Schritt 370).

Bei der erfindungsgemäßen Vorgehensweise ist zu beachten, dass die Anzahl m der aufzunehmenden Mess-Durchstrahlungsaufnahmen zwischen zwei Referenzzeitpunkten zum Aufnahmen einer oder mehrerer Referenz-Durchstrahlungsaufnahmen. d.h. die Abtastfrequenz der Referenz-Durchstrahlungsaufnahmen, über der Messdauer hinweg nicht konstant sein braucht. Beispielsweise könnte man die Anzahl m von Mess-Durchstrahlungsaufnahmen pro Teilsatz mit zunehmender Messdauer vergrößern oder dynamisch zur Laufzeit anhand der Ergebnisse der Vergleiche vorhergehender Referenz-Durchstrahlungsaufnahmen anpassen. In diesem Zusammenhang wird ferner darauf hingewiesen, dass mit der Aufnahme eines weiteren Teilsatzes von Mess-Durchstrahlungsaufnahmen des Untersuchungsobjekts 120 bereits begonnen werden kann, bevor der Ableitungsvorgang der Referenzinformationen über die Störeinflüsse abgeschlossen ist.

Ferner gibt die Anzahl der Erfassungsvorgänge für die Referenz-Durchstrahlungsaufnahmen zu unterschiedlichen Referenzzeitpunkten (Referenzzeitintervallen) ein Maß für die zeitliche Abtastung der Störeinflüsse bzw. der Änderung der Abbildungsgeometrie an. Die Reihenfolge der Erfassung bzw. Ermittlung der eigentlichen Mess-Durchstrahlungsaufnahme kann im Wesentlichen beliebig vorgenommen werden.

Beispielsweise kann die Aufnahme der Mess-Durchstrahlungsaufnahmen im Fall von n Stützstellen (d.h. n Referenzinformationen basierend auf den Referenz-Durchstrahlungsaufnahmen) zu n - 1 gleichen Teilen aufgeteilt werden, damit die zeitliche Abtastung der Stützstellen möglichst gleichmäßig über den Datensatz bzw. die Messzeit verteilt stattfindet. Bei dem vorliegenden Verfahren zur Datenermittlung stellt somit das Untersuchungsobjekt sein eigenes Referenzobjekt dar.

Das obige Verfahren 200, 300 zur Datenermittlung für eine Computertomographieaufnahme ist insbesondere für eine Kompensation von Auswirkungen von Brennfleckbewegungen bzw. Brennfleckwanderungen während der Erfassung von Mess-Durchstrahlungsaufnahmen des Untersuchungsobjekts 120 geeignet. Ist nun beispielsweise die CT-Anordnung in einer sogenannten Parallelstrahlgeometrie angeordnet, so führt eine Instabilität der Position der Röntgenstrahlenquelle, d.h. des Brennflecks, zu einer Translation der Abbildung des Untersuchungsobjekts 120. Mit Bildverarbeitungsmethoden kann diese Translation der Abbildung des Untersuchungsobjekts 120 aus ortsunveränderlichen Objektmerkmalen detektiert werden. Hierzu werden die oben bezeichneten Bildverarbeitungsmethoden auf die erfassten Referenz-Durchstrahlungsaufnahmen (gemäß der oben dargestellten Vorgehensweise) angewendet.

Bei einer Ausbildung der CT-Anordnung mit einer Fächer- oder Kegelstrahlgeometrie führt eine Bewegung des optischen Brennflecks nur näherungsweise zu einer Translation der Abbildung des Untersuchungsobjekts, da das Untersuchungsobjekt 120 aus einer anderen Perspektive durchstrahlt wird. Die Näherung ist dabei umso besser, je geringer die perspektivische Verzerrung durch die Bewegung des optischen Brennflecks bzw. je kleiner der durch den Fokus-Detektor-Abstand (FDA) und der Detektorbreite bzw. Detektorhöhe definierte Öffnungswinkel ist. Dieser Winkel verringert sich mit einem zunehmenden FDA.

Aus der Verschiebung der Objektabbildung kann zwar nicht auf die tatsächliche Position, jedoch auf die Bewegung des optischen Brennflecks geschlossen werden. Bei Ausführungsbeispielen der vorliegenden Erfindung zur Datenermittlung für eine Störeinfluss-reduzierte Computertomographieaufnahme sind keine speziellen Kalibrierkörper erforderlich.

Die Bildsequenz, d.h. die Referenz-Durchstrahlungsaufnahmen, zur Bestimmung der aus der Brennfleckbewegung resultierenden Abbildungsverschiebungen wird gemäß Ausführungsbeispielen durch ein mehrfaches Anfahren derselben Aufnahmegeometrie erzeugt, d.h. zu unterschiedlichen Referenzzeitpunkten wird jeweils dieselbe Aufnahmegeometrie mit einer oder mehreren Referenz-Durchstrahlungsaufnahmen erzeugt.

Aus dieser Bildsequenz der Referenz-Durchstrahlungsaufnahmen können beispielsweise die relativen Positionen des optischen Brennflecks zu diskreten Zeitpunkten t + ix bestimmt werden. Dabei stellt t den ersten Zeitpunkt eines Referenzzeitpunkts dar, während x die Zeitdauer zwischen zwei Referenzzeitpunkten darstellt. Diese zeitdiskreten Punkte stellen nun beispielsweise die Stützstellen für die Bestimmung einer im Hinblick auf Ort und Zeit stetigen Funktion (Korrekturfunktion) dar, welche die relative Position des optischen Brennflecks zu jedem Zeitpunkt des Messzeitraums zwischen den Referenzzeitpunkten approximativ beschreibt. Somit lässt sich anhand dieser Korrekturfunktion die relative Position des optischen Brennflecks für jede Mess-Durchstrahlungsaufnahme auf der Grundlage einer Interpolation herleiten. Die Güte der Herleitung kann dabei durch ein endliches Erhöhen der zeitlichen Abtastfrequenz der Referenz-Durchstrahlungsaufnahmen verbessert werden.

Erfindungsgemäß wird also eine (z.B. hinsichtlich Ort und Zeit stetige) Korrekturfunktion mittels einer Interpolation der als Stützstellen dienenden Referenzinformationen gebildet, wobei die Korrekturfunktion einen zeitlichen Verlauf bzw. eine zeitliche Abhängigkeit des Störeinflusses auf die Mess-Durchstrahlungsaufnahmen des Durchstrahlungsdatensatzes wiedergibt. Der Störeinfluss bzw. die Störgröße ist dabei die Änderung der Abbildungsgeometrie des CT-Systems aufgrund einer Brennfleckwanderung der Röntgenstrahlungsquelle des CT-Systems.

Falls nun der in Schritt 360 von Fig. 3 dargestellte Rekonstruktionsvorgang der zeitlich zwischen t und t + x aufgenommenen Mess-Durchstrahlungsaufnahmen basierend auf einem Rekonstruktionsalgorithmus durchgeführt wird, kann die Korrekturfunktion, welche die relative Brennfleckposition zu jedem Zeitpunkt im Messzeitraum beschreibt, dem Rekonstruktionsalgorithmus als eine, auf die Mess-Durchstrahlungsaufnahmen bezogene Zusatzinformation zur Verfügung gestellt werden. Somit kann der Rekonstruktionsvorgang unter Verwendung des Rekonstruktionsalgorithmus und unter Berücksichtigung der Korrekturfunktion, die eine zeitliche Änderung der Abbildungsgeometrie des CT-Systems wiedergibt, durchgeführt werden, um die Störeinfluss-kompensierte Computertomographieaufnahme basierend auf einer Kombination aller rekonstruierten und Störeinflusskompensierten Teilsätze der Mess-Durchstrahlungsaufnahmen zu erhalten. Somit kann der Tiefpasscharakter eines sich bewegenden Brennflecks in der Rekonstruktion der Mess-Durchstrahlungsaufnahmen kompensiert werden. Dies führt zu einer höheren Auflösung bzw. Detailerkennbarkeit in der CT-Aufnahme durch das Messsystem.

Eine Kombination aller rekonstruierten Teilsätze von Mess-Durchstrahlungsaufnahmen führt also zu der rekonstruierten Computertomographieaufnahme. Für den Fall, dass ein Rekonstruktionsalgorithmus zum Einsatz kommt, der keine Veränderung der Abbildungsgeometrie über der Messzeit berücksichtigen kann, besteht gemäß einem weiteren Ausführungsbeispiel eine Störeinfluss-Kompensation in einer Computertomographieaufnahme darin, einen modifizierten Durchstrahlungsdatensatz durch digitales Verschieben der mit einem Störeinfluss-behafteten Mess-Durchstrahlungsaufnahmen entgegengesetzt zu der erfassten Änderung der Abbildungsgeometrie basierend auf den Korrekturdaten oder der Korrekturfunktion durchzuführen, wobei dann die Computertomographieaufnahme basierend auf dem modifizierten Durchstrahlungsdatensatz rekonstruiert wird. Es ist also möglich, den modifizierten Durchstrahlungsdatensatz durch ein digitales Verschieben der Mess-Durchstrahlungsaufnahmen entgegengesetzt der detektierten Verschiebung der Referenz-Durchstrahlungsaufnahmen vorzunehmen. Dadurch kann der Einfluss der Bewegung des Brennflecks bereits auf Ebene der erfassten Daten der Mess-Durchstrahlungsaufnahmen näherungsweise invertiert werden. Der Rekonstruktionsalgorithmus kann dann den optischen Brennfleck als stationär voraussetzen. Aufgrund der oben beschriebenen Näherung wird jedoch im Falle einer Fächer- oder Kegelstrahlgeometrie die Kompensation während der Rekonstruktion als effektiv angesehen.

Im Folgenden werden nun sich ergebende Effekte (Wirkungen und/oder Vorteile) von Ausführungsbeispielen der vorliegenden Erfindung dargestellt. Die Ausführungsbeispiele der vorliegenden Erfindung bilden die Grundlage für die Detektion und Kompensation von zeitabhängigen Störeinflüssen bei einer Computertomographieaufnahme in einem Computertomographiesystem. Geometrische und andere (z.B. thermische) Einflussfaktoren können gemäß dem erfindungsgemäßen Konzept zur Datenermittlung für eine Computertomographieaufnahme eines Untersuchungsobjekts in einem CT-System und insbesondere dem Computertomographiesystem zuverlässig detektiert und quantifiziert werden.

Bei Ausführungsbeispielen der vorliegenden Erfindung werden unterschiedliche Teilsätze von Mess-Durchstrahlungsaufnahmen und Referenz-Durchstrahlungsaufnahmen abwechselnd zueinander erfasst, wobei Referenz-Durchstrahlungsaufnahmen an einer oder mehreren Referenzpositionen mehrfach aufgenommen werden, d.h. nacheinander zu den jeweiligen Referenzzeitpunkten. Dadurch kann eine frühzeitige Erkennung und Berücksichtigung von Störeinflüssen in der Bilddatenaufnahme erfolgen. Dies führt zu einer Verbesserung der Bildqualität durch Kompensation dieser frühzeitig erkannten Störeinflüsse, und somit gleichermaßen zu Verbesserungen von Prüf- und Analyseergebnissen durch die verbesserte Bildqualität. Ferner kann das erfindungsgemäß Konzept mit einer Zeitersparnis betrieben werden, da kein zusätzlicher vollständiger schneller Scan des Untersuchungsobjekts notwendig ist. Ferner können sehr gute Kompensationsergebnisse durch die zeitliche Korrelation zwischen den jeweiligen Referenz-Durchstrahlungsaufnahmen und den zugeordneten Mess-Durchstrahlungsaufnahmen erreicht werden sowie durch die Tatsache, dass bei der Aufnahme einer Referenz-Durchstrahlungsaufnahme bzw. einer geringen Anzahl von Referenz-Durchstrahlungsaufnahmen annähernd keine Brennfleckbewegung stattfindet, da es sich nur um eine einzige bzw. eine geringe Anzahl von Referenz-Durchstrahlungsaufnahmen handelt. Es können daher eine verbesserte Bildqualität der resultierenden 2D-/3D-Computertomographieaufnahme und auch präzisere und robustere Mess- und Analyseergebnisse erreicht werden.

Ferner sind keine iterativen Abläufe erforderlich, sondern es kann eine starke Parallelisierung in der Verarbeitung der Mess-Durchstrahlungsaufnahmen basierend auf den zugeordneten Referenz-Durchstrahlungsaufnahmen erfolgen. Insbesondere ist eine (sofortige) Rekonstruktion eines Teilsatzes der Mess-Durchstrahlungsaufnahmen nach Abschluss der nachfolgenden Referenz-Durchstrahlungsaufnahme und Ermittlung der Referenzinformationen möglich (es kann lediglich eine minimale Verzögerung höchstens um jeweils die Erfassungszeitdauer der m Mess-Durchstrahlungsaufnahmen erfolgen zzgl. der Dauer des Schritts 350). Ferner ergibt sich ein optimal ausgenutzter aktiver Bildbereich des Röntgendetektors 130, da keine Referenzobjekte erforderlich bzw. vorhanden sind und analysiert werden müssen, die den Bildbereich zumindest teilweise abschatten und nicht für eine Bildwiedergabe zur Verfügung stehen. Da gemäß Ausführungsbeispielen der vorliegenden Erfindung kein Referenzobjekt eingesetzt werden muss, ergibt sich ferner eine Zeitersparnis für den Gesamtablauf, da dessen Position bzw. Größe oder Beschaffenheit nicht an die geometrische Vergrößerung oder an die Strahlenhärte der eingesetzten Röntgenstrahlung angepasst werden muss.

Ferner gibt es praktisch keine (zusätzlichen) Anforderungen an das Manipulationssystem, d.h. an die Verarbeitungs- und Steuerungseinrichtung 170 und die zugeordneten Stellmotoren (nicht gezeigt in Fig. 1), da keine Verschiebung der Komponenten zur Kompensation der Bewegung des optischen Brennflecks bereits vor der Aufnahme der Mess-Durchstrahlungsaufnahmen erfolgt.

Gemäß Ausführungsbeispielen wirkt das Verfahren zur Datenermittlung derart auf die Datenakquisition aus, dass die Datenakquisition nicht nur genau eine Umdrehung (einen Vollumlauf) des Untersuchungsobjekts voraussetzt, sondern die Winkelpositionen in einer veränderten Reihenfolge in Form von n Umdrehungen oder beliebigen anderen Abtastreihenfolgen angefahren werden kann. Durch die Aufnahme von n' ≥ n Referenz-Durchstrahlungsaufnahmen ergibt sich eine sehr hohe zeitliche Abtastung zur Ermittlung der Brennfleckposition. Je nach Größe des gewählten Werts m (für die Anzahl von Mess-Durchstrahlungsaufnahmen pro Teilsatz) ist diese Vorgehensweise zwar mit einem erhöhten zeitlichen Aufwand verglichen mit einer reinen Messdatenakquisition verbunden, der maßgeblich von der Geschwindigkeit der Drehachse abhängt. Durch die erhöhte zeitliche Abtastung der Referenz-Durchstrahlungsaufnahmen können jedoch auch hochfrequente, d.h. sich sehr schnell ändernde Störfaktoren, wie z.B. die Brennfleckbewegungen der Röntgenstrahlungsquelle 110 erkannt und kompensiert werden.

Damit kann eine erhöhte Präzision der Kompensation der Brennfleckbewegung erreicht werden. Neben der Winkelabhängigkeit der Bestimmbarkeit der Brennfleckposition wird auch die zeitliche Komponente einbezogen, so dass abhängig von der Abtastgeschwindigkeit auch hochfrequente, d.h. sich relativ schnell ändernde Brennfleckbewegungen detektiert und kompensiert werden können. Dies führt unmittelbar zu einer reduzierten Unschärfe der resultierenden Computertomographieaufnahme, also zu einer verbesserten Bitqualität in den Rekonstruktionsdaten. Auf Basis der verbesserten Bilddaten können präzisere und robustere Analyse- und Messergebnisse entsprechend der jeweiligen Prüfaufgabe erzielt werden. Insbesondere können die oben beschriebenen Verbesserungen der Bildqualität auch ohne den Einsatz von Referenzkörpern erzielt werden. Es kann daher die maximale Bildgröße für die Abbildung des Untersuchungsobjekts benutzt werden, wodurch eine maximale Ortsauflösung und Detailerkennbarkeit realisiert werden können.

Über die Detektion und Kompensation einer Brennfleckbewegung hinaus kann ferner auch eine Veränderung der Vergrößerung in der Abbildung erkannt und kompensiert werden sowie unter Randbedingungen auch eine Bewegung des Objekts.

Das erfindungsgemäße Konzept zur Datenermittlung für eine Störeinfluss-reduzierte Computertomographieaufnahme eines Untersuchungsobjekts ist im Wesentlichen bei allen Computertomographiesystemen und insbesondere Computertomographiesystemen, mit denen hohe Ortsauflösungen (≤ 5 µm) erreicht werden sollen, einsetzbar. Die Verarbeitungs- und Steuerungseinrichtung 170 kann nun ausgebildet sein, um die Mess-Durchstrahlungsaufnahmen bzw. den daraus resultierenden vollständigen Durchstrahlungsdatensatz (d.h. die Originalbildinformationen) nicht zu verwerfen, nachdem eine Rekonstruktion des Durchstrahlungsdatensatzes unter Verwendung der Referenzinformationen durchgeführt wurde. Somit ist es jederzeit transparent nachvollziehbar, inwieweit eine Kompensation einer Brennfleckbewegung stattgefunden hat, wobei die Rekonstruktionsergebnisse mit und ohne Kompensation basierend auf den Referenzinformationen verglichen werden können.

Bei der Computertomographie können die Ausführungsbeispiele der vorliegenden Erfindung vor allem für die Kompensation von horizontalen und vertikalen Brennfleckwanderungen für die Kompensation von Vergrößerungsänderungen sowie für eine Kompensation von Schwankungen in der Strahlintensität oder für eine Detektion von Änderungen im Strahlungsspektrum verwendet werden. Hierzu sind keine zusätzlichen Anforderungen an existierenden CT-Systemen erforderlich als diese ohnehin für die Durchführung einer Computertomographieaufnahme vorhanden sind.

Im Fall von Radioskopiesystemen können Ausführungsbeispiele der vorliegenden Erfindung eingesetzt werden, indem statt Einzelaufnahmen mehrere Durchstrahlungsaufnahmen mit entsprechend kürzerer Belichtungszeit aufgenommen und anschließend aufsummiert werden. In diesem Fall ist keine Objektrotationsachse erforderlich. Damit sind Ausführungsbeispiele der vorliegenden Erfindung auch bei Röntgensystemen einsetzbar, die mit geringen Photonenflüssen arbeiten.

Ferner ist anzumerken, dass das erfindungsgemäße Konzept grundsätzlich nicht auf der Anwendung in der Röntgentechnik beschränkt ist, sondern für alle Bildgebungsverfahren, bei denen zeitabhängige Störeinflüsse vorkommen und möglichst kompensiert werden sollen, denkbar ist.

Obwohl manche Aspekte im Zusammenhang mit einer Bildverarbeitungsvorrichtung beschrieben wurden, versteht es sich, dass diese Aspekte auch eine Beschreibung des entsprechenden Verfahrens zur Ermittlung von Kalibrierdaten für ein ComputertomographieSystem darstellen, so dass ein Block oder ein Bauelement einer Vorrichtung auch als ein entsprechender Verfahrensschritt oder als ein Merkmal eines Verfahrensschritts zu verstehen ist. Analog dazu stellen Aspekte, die im Zusammenhang mit einem oder als ein Verfahrensschritt beschrieben wurden auch eine Beschreibung eines entsprechenden Blocks oder Details oder Merkmals einer entsprechenden Vorrichtung dar. Einige oder alle Verfahrensschritte können auch durch einen Hardware-Apparat (oder unter Verwendung eines Hardware-Apparats), wie z.B. einem Mikroprozessor, einem programmierbaren Computer oder eine elektronische Schaltung ausgeführt werden. Bei einigen Ausführungsbeispielen können einige oder mehrere der wichtigen Verfahrensschritte durch einen solchen Apparat ausgeführt werden.

Je nach bestimmtem Implementierungsaufwand können Ausführungsbeispiele der Erfindung in Hardware oder in Software, wie z.B. in der Volex Software, implementiert sein. Die Implementierung kann unter Verwendung eines digitalen Speichermediums, beispielsweise einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft. Der Programmcode kann beispielsweise auch auf einem maschinenlesbaren Träger gespeichert sein. Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist. Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen. Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Verfahren (200; 300) zur Datenermittlung für eine Computertomographieaufnahme eines Untersuchungsobjekts (120), mit folgenden Schritten:
Erfassen (210) einer Abfolge unterschiedlicher Teilsätze von Mess-Durchstrahlungsaufnahmen des Untersuchungsobjekts (120) an vorgegebenen, unterschiedlichen Mess-Winkelpositionen, um basierend auf den unterschiedlichen Teilsätzen von Mess-Durchstrahlungsaufnahmen eine Gesamtzahl von Mess-Durchstrahlungsaufnahmen zu erhalten;
wiederholtes Erfassen (220) einer Referenz-Durchstrahlungsaufnahme des Untersuchungsobjekts (120) zu unterschiedlichen Referenzzeitpunkten an derselben Referenz-Winkelposition, wobei jeweils ein Referenzzeitpunkt der unterschiedlichen Referenzzeitpunkte zeitlich zwischen zwei Erfassungsvorgängen (210) für die unterschiedlichen Teilsätze von Mess-Durchstrahlungsaufnahmen liegt; und
Ermitteln (350) von Referenzinformationen basierend auf den Referenz-Durchstrahlungsaufnahmen, wobei die Referenzinformationen einen Störeinfluss auf die Mess-Durchstrahlungsaufnahmen jeweils zu den unterschiedlichen Referenzzeitpunkten angeben.

2. Verfahren nach Anspruch 1, mit folgendem Schritt:
Rekonstruieren (370) der Computertomographieaufnahme basierend auf der Gesamtzahl von Mess-Durchstrahlungsaufnahmen und den Referenzinformationen, um eine Störeinfluss-kompensierte Computertomographieaufnahme des Untersuchungsobjekts (120) zu erhalten.

3. Verfahren nach einem der Ansprüche 1 oder 2, wobei bei dem Schritt des Erfassens einer Referenz-Durchstrahlungsaufnahme zu einem Referenzzeitpunkt eine weitere oder eine Mehrzahl von weiteren Referenz-Durchstrahlungsaufnahmen des Untersuchungsobjekts an unterschiedlichen, vorgegebenen Referenz-Winkelpositionen zu unterschiedlichen, vorgegebenen Referenz-Zeitpunkten erfasst werden, um die Referenzinformationen zu ermitteln.

4. Verfahren nach einem der vorhergehenden Ansprüche, wobei bei dem Schritt (350) des Ermittelns der Referenzinformationen ein Bildverarbeitungsvorgang an den Referenz-Durchstrahlungsaufnahmen durchgeführt wird.

5. Verfahren nach einem der vorhergehenden Ansprüche, mit folgendem Schritt:
Ermitteln (340) einer Korrekturfunktion mittels einer Interpolation der als Stützstellen dienenden Referenzinformationen, wobei die Korrekturfunktion einen zeitlichen Verlauf des Störeinflusses auf die Mess-Durchstrahlungsaufnahmen wiedergibt.

6. Verfahren nach einem der vorhergehenden Ansprüche, mit folgendem Schritt:
Ermitteln der Referenz-Winkelposition für den Erfassungsvorgang der Referenz-Durchstrahlungsaufnahmen des Untersuchungsobjekts (120) basierend auf einer geometrischen Form oder einem Aspektverhältnis des Untersuchungsobjekts, um an der ermittelten Referenz-Winkelposition basierend auf einer resultierenden Außenkontur, einem resultierenden Bildkontrast, resultierenden Durchstrahlungslängen und/oder resultierenden Innenstrukturen aus den Referenz-Durchstrahlungsaufnahmen ableitbare Informationen hinsichtlich des Störeinflusses auf die Mess-Durchstrahlungsaufnahmen zu erhalten.

7. Verfahren nach einem der vorhergehenden Ansprüche, wobei der erste Teilsatz von Mess-Durchstrahlungsaufnahmen mit einer Mess-Schrittweite, zwischen 5° und 30°erfasst wird; wobei die Referenz-Durchstrahlungsaufnahme des Untersuchungsobjekts (120) an einer Referenz-Winkelposition zu einem Referenzzeitpunkt erfasst wird, um die Referenzinformation zu erhalten; und wobei bei dem wiederholten Erfassen (220) einer Referenz-Durchstrahlungsaufnahme ein weiterer Teilsatz von Mess-Durchstrahlungsaufnahmen mit einer weiteren Mess-Schrittweite erfasst wird, die zu der Mess-Schrittweite unterschiedlich ist oder dazu versetzte Mess-winkelpositionen vorgibt, wobei die weitere Mess-Schrittweite zwischen 5° und 30° liegt; ferner mit folgenden Schritten:
Erfassen einer weiteren Referenz-Durchstrahlungsaufnahme an der Referenz-Winkelposition, um eine weitere Referenzinformation zu dem weiteren Referenz-Erfassungszeitpunkt zu erhalten; und
Wiederholen der obigen Schritte des Erfassens eines weiteren Teilsatzes von Mess-Durchstrahlungsaufnahmen und von weiteren Referenz-Durchstrahlungsaufnah-men, bis die Gesamtzahl von Mess-Durchstrahlungsaufnahmen und die zugehörigen Referenzinformationen vorliegen.

8. Verfahren nach einem der Ansprüche 5 bis 7, mit folgendem Schritt:
Durchführen des Rekonstruktionsvorgangs (360) unter Verwendung eines Rekonstruktionsalgorithmus und unter Berücksichtigung der Korrekturfunktion, die einen zeitlichen Verlauf des Störeinflusses auf die Mess-Durchstrahlungsaufnahmen und somit eine zeitliche Änderung der Abbildungsgeometrie des Computertomographiesystems wiedergibt, um die Störeinfluss-kompensierte Computertomographieaufnahme zu erhalten.

9. Verfahren nach einem der Ansprüche 5 bis 8, mit folgendem Schritt:
Erstellen eines modifizierten Durchstrahlungsdatensatzes basierend auf der Gesamtzahl von Mess-Durchstrahlungsaufnahmen durch digitales Verschieben der mit dem Störeinfluss-behafteten Mess-Durchstrahlungsaufnahmen entgegengesetzt zu einer erfassten Änderung der Abbildungsgeometrie basierend auf der Korrekturfunktion.

10. Verfahren nach Anspruch 9, mit folgendem Schritt:
Rekonstruieren der Computertomographieaufnahme basierend auf dem modifizierten Durchstrahlungsdatensatz.

11. Computerprogramm mit einem Programmcode zur Durchführung der Verfahrensschritte nach einem der Ansprüche 1 bis 10, wenn das Computerprogramm auf einem Computer abläuft.

12. Computertomographiesystem (100) zur Datenermittlung für eine Computertomographieaufnahme eines Untersuchungsobjekts (120), mit folgenden Merkmalen:
einer Computertomographieanordnung (110, 130) zum Erstellen von Durchstrahlungsaufnahmen eines Untersuchungsobjekts (120), wobei die Computertomographieanordnung (110, 130) und das Untersuchungsobjekt (120) relativ zueinander rotierbar angeordnet sind; und
einer Verarbeitungs- und Steuerungseinrichtung (170), die mit der Computertomographieanordnung (110, 130) gekoppelt ist und ausgebildet ist, um eine Abfolge unterschiedlicher Teilsätze von Mess-Durchstrahlungsaufnahmen des Untersuchungsobjekts (120) an vorgegebenen Winkelpositionen zu erfassen, um basierend auf den unterschiedlichen Teilsätzen von Mess-Durchstrahlungsaufnahmen eine Gesamtzahl von Mess-Durchstrahlungsaufnahmen zu erhalten, um eine Referenz-Durchstrahlungsaufnahme des Untersuchungsobjekts zu unterschiedlichen Referenzzeitpunkten an derselben Referenz-Winkelposition wiederholt zu erfassen, wobei jeweils einer der unterschiedlichen Referenzzeitpunkte zeitlich zwischen zwei Erfassungsvorgängen für die unterschiedlichen Teilsätze von Mess-Durchstrahlungsaufnahmen liegt;
wobei die Verarbeitungs- und Steuerungseinrichtung (170) ferner ausgebildet ist,
um basierend auf den Referenz-Durchstrahlungsaufnahmen Referenzinformationen zu ermitteln, wobei die Referenzinformationen den Störeinfluss auf die Mess-Durchstrahlungsaufnahmen jeweils zu den unterschiedlichen Referenzzeitpunkten angeben.

13. Computertomographiesystem gemäß Anspruch 12, wobei die Verarbeitungs- und Steuerungseinrichtung (170) ausgebildet ist, um die Computertomographieaufnahme basierend auf der Gesamtzahl von Mess-Durchstrahlungsaufnahmen und den Referenzinformationen zu rekonstruieren, um eine Störeinfluss-kompensierte Computertomographieaufnahme des Untersuchungsobjekts zu erstellen.

14. Computertomographiesystem nach einem der Ansprüche 12 oder 13, wobei die Computertomographieanordnung eine Röntgenrückstrahlungsquelle (110) zur Erzeugung einer das Untersuchungsobjekt (120) durchdringenden Röntgenstrahlung, und einen für Röntgenstrahlung empfindlichen Detektor (130) zum Erfassen der Durchstrahlungsaufnahmen des Untersuchungsobjekts (120) aufweist.

15. Computertomographiesystem nach einem der Ansprüche 12 bis 14, wobei die Verarbeitungs- und Steuerungseinrichtung (170) ferner ausgebildet ist, um die Verfahrensschritte nach einem der Ansprüche 4 bis 13 durchzuführen.

## Claims

1. A method (200; 300) for data determination for a computer tomography recording of a test object (120), comprising:
detecting (210) a sequence of different subsets of measurement transmission recordings of the test object (120) at predetermined, different measurement angle positions to acquire an overall number of measurement transmission recordings based on the different subsets of measurement transmission recordings; and
determining (350) reference information based on the reference transmission recordings,
wherein the reference information indicates an interference on the measurement transmission recordings each at the different reference times.

2. The method according to claim 1, further comprising:
reconstructing (370) the computer tomography recording based on the overall number of measurement transmission recordings and the reference information in order to acquire an interference-compensated computer tomography recording of the test object (120).

3. The method according to one of claims 1 or 2, wherein in the step of detecting a reference transmission recording at a reference time one further or a plurality of further reference transmission recordings of the test object are detected at different, predetermined reference angle positions in order to determine the reference information.

4. The method according to one of the preceding claims, wherein in step (350) of determining the reference information an image processing operation is executed on the reference transmission recordings.

5. The method according to one of the preceding claims, further comprising:
determining (340) a correction function using an interpolation of the reference information serving as supporting points, wherein the correction function reproduces a temporal course of the interference with respect to the measurement transmission recordings.

6. The method according to one of the preceding claims, comprising:
determining the reference angle position for the detection process of the reference transmission recordings of the test object (120) based on a geometrical shape or an aspect ratio of the test object in order to acquire, at the determined reference angle position, based on a resulting exterior contour, a resulting image contrast, resulting transmission lengths and/or resulting interior structures, information derivable from the reference transmission recording with regard to the interference on the measurement transmission recordings.

7. The method according to one of the preceding claims, wherein the first subset of measurement transmission recordings is detected with a measurement step width between 5° and 30°; wherein the reference transmission recording of the test object (120) is detected at a reference angle position at a reference time in order to acquire the reference information; and wherein, when repeatedly detecting (220) a reference transmission recording, a further subset of measurement transmission recordings is detected with a further measurement step width which is different from the measurement step width or presets measurement angle position offset from the same, wherein the further measurement step width lies between 5° and 30°; further comprising:
detecting a further reference transmission recording at the reference angle position in order to acquire further reference information with respect to the further reference detection time; and
repeating the above steps of detecting a further subset of measurement transmission recordings and further reference transmission recordings until the complete number of measurement transmission recordings and the associated reference information are present.

8. The method according to one of claims 5 to 7, comprising:
executing the reconstruction process (360) using a reconstruction algorithm and considering the correction function which reproduces a temporal course of the interference on the measurement transmission recordings and thus a temporal change of the image geometry of the computer tomography system in order to acquire the interference-compensated computer tomography recording.

9. The method according to one of claims 5 to 8, further comprising:
generating a modified transmission dataset based on the overall number of measurement transmission recordings by digitally shifting the measurement transmission recordings containing interferences opposite to the detected change of the image geometry based on the correction function.

10. The method according to claim 9, comprising:
reconstructing the computer tomography recording based on the modified transmission dataset.

11. A computer program having a program code for executing the method steps according to one of claims 1 to 10, when the computer program is executed on a computer.

12. A computer tomography system (100) for data determination for a computer tomography recording of a test object (120), comprising:
a computer tomography arrangement (110, 130) for generating transmission recordings of a test object (120), wherein the computer tomography arrangement (110, 130) and the test object (120) are arranged rotatably relative to each other; and
a processing and control means (170) which is coupled to the computer tomography arrangement (110, 130) and which is further implemented to detect a sequence of different subsets of measurement transmission recordings of the test object (120) at predetermined angle positions in order to acquire, based on the different subsets of measurement transmission recordings, an overall number of measurement transmission recordings to repeatedly detect a reference transmission recording of the test object at different reference times at the same reference angle position, wherein one of the different reference times each is temporally between two detection operations for the different subsets of measurement transmission recordings;
wherein the processing and control means (170) is further implemented to determine reference information based on the reference transmission recordings, wherein the reference information indicates the interference on the measurement transmission recordings at the different reference times each.

13. The computer tomography system according to claim 12, wherein the processing and control means (170) is implemented to reconstruct the computer tomography recording based on the overall number of measurement transmission recordings and the reference information in order to generate an interference-compensated computer tomography recording of the test object.

14. The computer tomography system according to one of claims 12 or 13, wherein the computer tomography arrangement comprises an x-ray reflection source (110) for generating x-rays penetrating the test object (120), and a detector (130) which is sensitive for x-rays for detecting the transmission recordings of the test object (120).

15. The computer tomography system according to one of claims 12 to 14, wherein the processing and control means (170) is further implemented to perform the method steps according to one of claims 4 to 13.

## Revendications

1. Procédé (200; 300) de détermination de données pour un enregistrement de tomographie assistée par ordinateur d'un objet à examiner (120), aux étapes suivantes consistant à:
détecter (210) une séquence de différents sous-ensembles d'enregistrements radiographiques de mesure de l'objet à examiner (120) dans différentes positions angulaires de mesure prédéterminées, pour obtenir, sur base des différents sous-ensembles de d'enregistrements radiographiques de mesure, un nombre total d'enregistrements radiographiques de mesure;
détecter de manière répétée (220) un enregistrement radiographique de référence de l'objet à examiner (120) à différents moments de référence dans la même position angulaire de référence, un moment de référence parmi les différents moments de référence se situant dans le temps entre deux opérations de détection (210) pour les différents sous-ensembles d'enregistrements radiographiques de mesure; et
déterminer (350) des informations de référence sur base des enregistrements radiographiques de référence, les informations de référence indiquant une influence parasite sur les enregistrements radiographiques de mesure respectivement aux différents moments de référence.

2. Procédé selon la revendication 1, à l'étape suivante consistant à:
reconstruire (370) l'enregistrement de tomographie assistée par ordinateur sur base du nombre total d'enregistrements radiographiques de mesure et des informations de référence, pour obtenir un enregistrement de tomographie assistée par ordinateur à influence parasite compensée de l'objet à examiner (120).

3. Procédé selon l'une des revendications 1 ou 2, dans lequel à l'étape de détection d'un enregistrement radiographique de référence à un moment de référence sont détectés un autre ou une pluralité d'autres enregistrements radiographiques de référence de l'objet à examiner en différentes positions angulaires de référence prédéterminées à différents moments de référence prédéterminés, pour déterminer les informations de référence.

4. Procédé selon l'une des revendications précédentes, dans lequel à l'étape (350) consistant à déterminer les informations de référence est effectuée une opération de traitement d'image sur les enregistrements radiographiques de référence.

5. Procédé selon l'une des revendications précédentes, à l'étape suivante consistant à:
déterminer (340) une fonction de correction au moyen d'une interpolation des informations de référence servant de points d'appui, la fonction de correction représentant une évolution dans le temps de l'influence parasite sur les enregistrements radiographiques de mesure.

6. Procédé selon l'une des revendications précédentes, à l'étape suivante consistant à:
déterminer la position angulaire de référence pour l'opération de détection des enregistrements radiographiques de référence de l'objet à examiner (120) sur base d'une forme géométrique ou d'un rapport d'aspect de l'objet à examiner, pour obtenir à la position angulaire de référence déterminée, sur base d'un contour extérieur résultant, d'un contraste d'image résultant, des longueurs d'irradiation résultantes et/ou des structures internes résultantes, des informations dérivables des enregistrements radiographiques de référence sur influence parasite sur les enregistrements radiographiques de mesure.

7. Procédé selon l'une des revendications précédentes, dans lequel le premier sous-ensemble d'enregistrements radiographiques de mesure est détecté avec un pas de mesure compris entre 5° et 30°; dans lequel l'enregistrement radiographique de référence de l'objet à examiner (120) est détecté dans une position angulaire de référence à un moment de référence, pour obtenir l'information de référence; et dans lequel lors de la détection répétée (220) d'un enregistrement radiographique de référence est détecté un autre sous-ensemble d'enregistrements radiographiques de mesure avec un autre pas de mesure qui est différent du pas de mesure ou prédétermine des positions angulaires décalées, l'autre pas de mesure étant compris entre 5° et 30°; par ailleurs aux étapes suivantes consistant à:
détecter un autre enregistrement radiographique de référence dans la position angulaire de référence, pour obtenir une autre information de référence à l'autre moment de détection de référence; et
répéter les étapes ci-dessus de détection d'un autre sous-ensemble d'enregistrements radiographiques de mesure et
d'autres enregistrements radiographiques de référence jusqu'à ce que soient présents le nombre total d'enregistrements radiographiques de mesure et les informations de référence y relatives.

8. Procédé selon l'une des revendications 5 à 7, avec l'étape suivante consistant à:
effectuer l'opération de reconstruction (360) à l'aide d'un algorithme de reconstruction, et en tenant compte de la fonction de correction qui représente une évolution dans le temps de l'influence parasite sur les enregistrements radiographiques de mesure et, donc, une variation dans le temps de la géométrie de reproduction du système de tomographie assistée par ordinateur,
pour obtenir l'enregistrement de tomographie assistée par ordinateur à influence parasite compensée.

9. Procédé selon l'une des revendications 5 à 8, à l'étape suivante consistant à:
établir un ensemble de données d'irradiation modifié sur base du nombre total d'enregistrements radiographiques de mesure par déplacement numérique des enregistrements radiographiques de mesure soumis à l'influence parasite contre une variation détectée de la géométrie de reproduction sur base de la fonction de correction.

10. Procédé selon la revendication 9, à l'étape suivante consistant à:
reconstruire l'enregistrement de tomographie assistée par ordinateur sur base de l'ensemble de données d'irradiation modifié.

11. Programme d'ordinateur avec un code de programme pour réaliser les étapes de procédé selon l'une des revendications 1 à 10 lorsque le programme d'ordinateur est exécuté sur un ordinateur.

12. Système de tomographie assistée par ordinateur (100) pour déterminer des données pour un enregistrement de tomographie assistée par ordinateur d'un objet à examiner (120), aux caractéristiques suivantes:
un aménagement de tomographie assistée par ordinateur (110, 130) destiné à réaliser des enregistrements radiographiques d'un objet à examiner (120), l'aménagement de tomographie assistée par ordinateur (110, 130) et l'objet à examiner (120) étant disposés de manière rotative l'un par rapport à l'autre; et
un moyen de traitement et de commande (170) qui est couplé à l'aménagement de tomographie assistée par ordinateur (130 110) et qui est réalisé pour détecter une séquence de différents sous-ensembles d'enregistrements radiographiques de mesure de l'objet à examiner (120) à des positions angulaires prédéterminées, pour obtenir, sur base des différents sous-ensembles d'enregistrements radiographiques de mesure, un nombre total d'enregistrements radiographiques de mesure, pour détecter de manière répétée un enregistrement radiographique de référence de l'objet à examiner à différents moments de référence dans la même position angulaire de référence, l'un respectif des différents moments de référence se situant dans le temps entre deux opérations de détection pour les différents sous-ensembles d'enregistrements radiographiques de mesure;
dans lequel l'aménagement de traitement et de commande (170) est par ailleurs réalisé pour déterminer, sur base des enregistrements radiographiques de référence, des informations de référence, où les informations de référence indiquent l'influence parasite sur les enregistrements radiographiques de mesure respectivement aux différents moments de référence.

13. Système de tomographie assistée par ordinateur selon la revendication 12, dans lequel le moyen de traitement et de commande (170) est configuré pour reconstruire l'enregistrement de tomographie assistée par ordinateur sur base du nombre total d'enregistrements radiographiques de mesure et des informations de référence, pour réaliser un enregistrement de tomographie assistée par ordinateur à influence parasite compensée de l'objet à examiner.

14. Système de tomographie assistée par ordinateur selon l'une des revendications 12 ou 13, dans lequel l'aménagement de tomographie assistée par ordinateur présente une source de rétro-réflexion de rayons X (110) destinée à générer un rayonnement de rayons X pénétrant dans l'objet à examiner (120) et un détecteur sensible aux rayons X (130) destiné à détecter les enregistrements radiographiques de l'objet à examiner (120).

15. Système de tomographie assistée par ordinateur selon l'une des revendications 12 à 14, dans lequel le moyen de traitement et de commande (170) est par ailleurs réalisé pour effectuer les étapes de procédé selon l'une des revendications 4 à 13.
